# EUROPEAN PATENT APPLICATION

(11) **EP 1 878 440 A1**
(43) Date of publication of application: **16.01.2008**
(21) Application number: 06291146.6
(22) Date of filing: 13.07.2006
(51) Int. Cl.: A61K 39/395, A61K 31/663, A61P 13/12, A61P 35/00, A61P 37/00

(54) **Methods and compositions for increasing the efficiency of therapeutic antibodies using gamma delta cell activator compounds**

(71) Applicant: INSERM (Institut National de la Santé et de la Recherche Médicale), 75654 Paris Cedex 13 (FR)
(72) Inventor: Laurent, Guy, F-31400 Toulouse (FR); Bezombes, Christine, F-31430 Le Fousseret (FR); Fournie, Jane Jacques, F-31450 Corronsac (FR); Gertner, Julie, F-31300 Toulouse (FR)
(74) Representative: Gallois, Valérie

(57) **Abstract**

The present invention relates to methods and compositions for increasing the efficiency of therapeutic antibodies. More particularly, the invention relates to the use of a therapeutic antibody in combination with a γδ T cell activating compound or activated γδ T cells, thereby allowing a potentiation of γδ T cell cytotoxicity in mammalian subjects in order to enhance the efficiency of the treatment in human subjects, particularly through an increase of the depletion of targeted cells.

## Description

### Field of the Invention

The present invention relates, generally, to methods and compositions for increasing the efficiency of therapeutic antibodies.

### Background of the Invention

Various therapeutic strategies in human beings are based on the use of therapeutic antibodies. These include, for instance, the use of therapeutic antibodies developed to deplete target cells, particularly diseased cells such as virally-infected cells, tumor cells or other pathogenic cells. Such antibodies are typically monoclonal antibodies, of IgG species, typically with human IgG1 or IgG3 Fc portions. These antibodies can be native or recombinant antibodies, and are often "humanized" mouse antibodies (i.e. comprising functional domains from various species, typically an Fc portion of human or non human primate origin, and with a variable region or complementary determining region (CDR) of mouse origin). Alternatively, the monoclonal antibody can be fully human through immunization in transgenic mice having the human Ig locus, or obtained through cDNA libraries derived from human cells.

A particular example of such therapeutic antibodies is rituximab (Mabthera^{®}, Rituxan^{®}), which is a chimeric anti-CD20 monoclonal antibody made with human γ1 and κ constant regions (therefore with human IgG1 Fc portion) linked to murine variable domains conferring CD20 specificity. In the past few years, rituximab has considerably modified the therapeutical strategy against B lymphoproliferative malignancies, particularly non-Hodgkin's lymphomas (NHL). Other examples of humanized IgG1 antibodies include alemtuzumab (Campath-1H^{®}), which is used in the treatment of B cell malignancies, and trastuzumab (Herceptin^{®}), which is used in the treatment of breast cancer. Additional examples of therapeutic antibodies under development are disclosed in the art.

The mechanism of action of therapeutic antibodies is still a matter of debate. Injection of antibodies leads to depletion of cells bearing the antigen specifically recognized by the antibody. This depletion can be mediated through at least three mechanisms: antibody mediated cellular cytotoxicity (ADCC), complement dependent lysis, and direct antitumor inhibition of tumor growth through signals given via the antigen targeted by the antibody.

While these antibodies represent a novel and efficient approach to human therapy, particularly for the treatment of tumors, they do not always exhibit a strong efficacy. For instance, while rituximab, alone or in combination with chemotherapy, was shown to be effective in the treatment of both low-intermediate and high-grade NHL, 30% to 50% of patients with low grade NHL have no clinical response to rituximab. It has been suggested that the level of CD20 expression on lymphoma cells, the presence of high tumor burden at the time of treatment, or low serum rituximab concentrations may explain the lack of efficacy of rituximab in some patients. Nevertheless, the actual causes of treatment failure remain largely unknown.

Further, the use of therapeutic antibodies can be limited by side effects caused by their administration. For example, side effects such as fever, headaches, nausea, hypotension, wheezing, rashes, infections, and numerous others can appear in patients, potentially limiting the possible amount or frequency with which the antibodies can be administered.

Thus, it would be very advantageous to increase the efficacy of therapeutic antibodies, or to be able to achieve therapeutic efficacy using reduced doses of the antibodies that are less likely to produce side effects. The present invention addresses these and other needs.

### Summary of the Invention

The present invention discloses novel approaches to enhance the efficacy of therapeutic antibodies. Indeed, the present invention provides novel compositions and methods that overcome the current difficulty related to the efficacy of therapeutic antibodies, particularly those intended to deplete a target cell (e.g. tumor cell, infected cell, inflammation-mediating cell, etc.). It is shown in the present invention that Vγ9δ2 T cells from an individual can effect the efficiency of therapeutic mAb (monoclonal antibody) when γδ T cells are activated and/or expanded, pointing to a synergy of therapeutic antibodies and γδ T cell activator therapies. Such conjoint use of a γδ T cell activator and therapeutic antibody leads to greater target cell lysis than observed using the therapeutic antibody along. It has also been observed that activation and/or expansion of γδ T cells can re-establish target cell lysis in cases where very little or no lysis was observed prior to activation and/or expansion of γδ T cells.

Therefore, the invention discloses methods of treatments of a subject in which a γδ T cell activator compound is co-administered with the therapeutic antibody to the subject. The inventors demonstrate here that the efficiency of a therapeutic antibody can be greatly enhanced by the co-administration, e.g., co-injection, of such a γδ T cell activator compound, preferably a phosphoantigen, that activates cell responses such as lysis, cytokine release and/or induces the proliferation of these cells. Optionally the phosphoantigen is further administered in conjunction with a cytokine, for example IL-2.

The present invention concerns a pharmaceutical composition comprising a γδ T cell activator compound, a therapeutic antibody and a pharmaceutically acceptable carrier. The present invention also concerns a kit or a product containing a γδ T cell activator compound and a therapeutic antibody as a combined preparation for simultaneous, separate or sequential use in the treatment of a disease.

The present invention also concerns the use of a γδ T cell activator compound and a therapeutic antibody for the preparation of a medicament for treating a disease.

The invention also concerns the use of a γδ T cell activator compound for increasing the efficiency of a treatment with a therapeutic antibody. In particular, the present invention concerns the use of a γδ T cell activator compound for the preparation of a drug for increasing the efficiency of a treatment involving the administration of a therapeutic antibody in a subject, wherein the administration of said therapeutic antibody is administered to said subject prior to, simultaneously with, or following, a therapeutically-effective amount of a γδ T cell activator compound.

In a particular aspect, the present invention provides a method of treatment of a disease in a human subject in need thereof, comprising : a) administering to said subject a γδ T cell activator compound; and, b) administering to said subject a therapeutic antibody.

Preferably, the therapeutic antibody targets diseased cells such as virally-infected cells, tumor cells, cells underlying an autoimmune disorder or other pathogenic cells. It can be a monoclonal, human, humanized or chimeric antibody or an antigen binding fragment thereof. In a preferred embodiment, the therapeutic antibody is rituximab or campath.

Preferably, the γδ T cell activator is a compound of formula (I) : 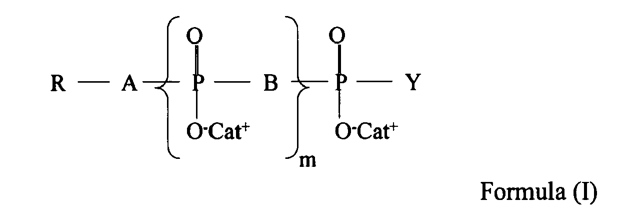
wherein Cat+ represents one (or several, identical or different) organic or mineral cation(s) (including proton);
m is an integer from 1 to 3;
B is O, NH, or any group capable to be hydrolyzed;
Y = O⁻Cat+, a C₁-C₃ alkyl group, a group -A-R, or a radical selected from the group consisting of a nucleoside, an oligonucleotide, a nucleic acid, an amino acid, a peptide, a protein, a monosaccharide, an oligosaccharide, a polysaccharide, a fatty acid, a simple lipid, a complex lipid, a folic acid, a tetrahydrofolic acid, a phosphoric acid, an inositol, a vitamin, a co-enzyme, a flavonoid, an aldehyde, an epoxyde and a halohydrin;
A is O, NH, CHF, CF₂ or CH₂; and,
R is a linear, branched, or cyclic, aromatic or not, saturated or unsaturated, C₁-C₅₀ hydrocarbon group, optionally interrupted by at least one heteroatom, wherein said hydrocarbon group comprises an alkyl, an alkylenyl, or an alkynyl, preferably an alkyl or an alkylene, which can be substituted by one or several substituents selected from the group consisting of : an alkyl, an alkylenyl, an alkynyl, an epoxyalkyl, an aryl, an heterocycle, an alkoxy, an acyl, an alcohol, a carboxylic group (-COOH), an ester, an amine, an amino group (-NH₂), an amide (-CONH₂), an imine, a nitrile, an hydroxyl (-OH), a aldehyde group (-CHO), an halogen, an halogenoalkyl, a thiol (-SH), a thioalkyl, a sulfone, a sulfoxide, and a combination thereof.

In a more preferred embodiment, the γδ T cell activator is a compound of formula (II): 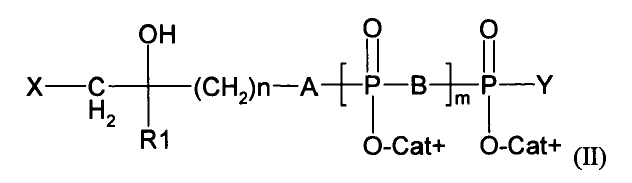 in which X is an halogen (preferably selected from I, Br and Cl), B is O or NH, m is an integer from 1 to 3, R1 is a methyl or ethyl group, Cat+ represents one (or several, identical or different) organic or mineral cation(s) (including the proton), and n is an integer from 2 to 20, A is O, NH, CHF, CF₂ or CH₂, and Y is O⁻Cat+.

In particular, the γδ T cell activator can be BrHPP, C-BrHPP or N-BrHPP.In an other more preferred embodiment, the γδ T cell activator is a compound of formula (XII): 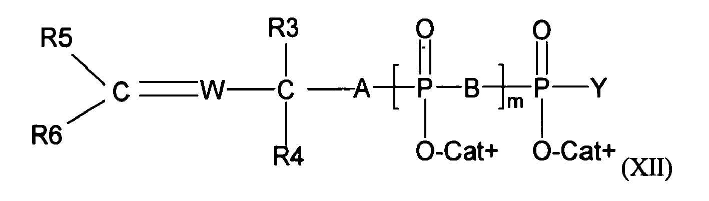 in which R₃, R₄, and R₅, identical or different, are a hydrogen or (C₁-C₃)alkyl group, W is -CH- or -N-, R₆ is an (C₂-C₃)acyl, an aldehyde, an (C₁-C₃)alcohol, or an (C₂-C₃)ester, Cat+ represents one (or several, identical or different) organic or mineral cation(s) (including the proton), B is O or NH, m is an integer from 1 to 3, A is O, NH, CHF, CF₂ or CH₂, and Y is O⁻Cat+.

In particular, the γδ T cell activator can be HDMAPP, C-HDMAPP or N-HDMAPP.

Preferably, the γδ T cell activator can also be an aminophosphonate of formula XVII : 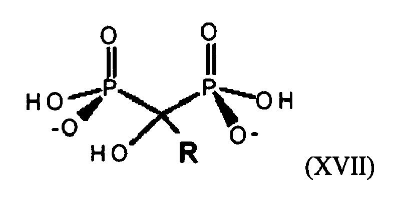 with R being a linear, branched, or cyclic, aromatic or not, saturated or unsaturated, C₁-C₅₀ hydrocarbon group, wherein said hydrocarbon group comprises an alkyl, an alkylenyl, or an alkynyl, preferably an alkyl or an alkylene, which is substituted by one or several substituents selected from the group consisting of : an amine, an amino group (-NH₂), an amide (-CONH₂), an imine, and a combination thereof.

In a preferred embodiment, R of formula XVII is a linear, branched, or cyclic, aromatic or not, saturated or unsaturated, C₁-C₁₀ hydrocarbon group, which is substituted by an amine, an amino group, a pyridine group, a pyrimidine group, a pyrrole group, an imidazole group, a pyrazole group, a triazole group.

In a still more preferred embodiment, R of formula XVII is selected from the group consisting of :

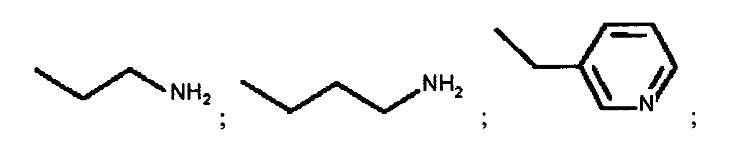

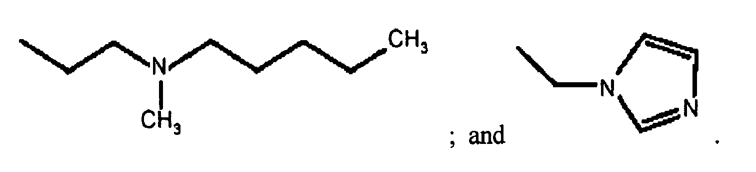

In particular, the γδ T cell activator can be selected from the group consisting of pamidronate, alendronate, ibandronate, risedronate and zoledronate.

In a particular embodiment, the disease requires the depletion of the targeted cells, preferably the diseased cells such as virally-infected cells, tumor cells, cells underlying an autoimmune disorder or other pathogenic cells. The disease can be selected from the group consisting of a cancer, an auto-immune disease, an inflammatory disease, and an infectious (e.g. bacterial or viral) disease. he disease also concerns a graft rejection, more particularly allograft rejection, and graft versus host disease (GVHD).

### Brief Description of the Drawings

FIGURE 1: Phosphoantigen activator Picostim plus anti-CD20 Rituximab increase the killing of B- cell lymphoma representative of Mantle Cell Lymphoma (see cell lines GRANTA, NCEB1).
FIGURE 2: Phosphoantigen activator Picostim plus anti-CD20 Rituximab increase the killing of B- cell lymphoma representative of Follicular lymphoma (see cell lines RL and KARPAS-422).
FIGURE 3: Phosphoantigen activator Picostim plus anti-CD20 Rituximab increase the killing of B- cell lymphoma representative of Burkitt Lymphoma (see cell lines Raji and DAUDI).
FIGURE 4: Phosphoantigen activator Picostim plus anti-CD52 Trastuzumab (Campath) increase the killing of B- cell lymphoma representative of Mantle Cell Lymphoma (see cell lines ES-MOULT, GRANTA).
FIGURE 5: Phosphoantigen activator Picostim plus anti-CD52 Trastuzumab increase the killing of B- cell lymphoma representative of Follicular lymphoma (see cell lines RL).
FIGURE 6: Phosphoantigen activator Picostim plus anti-CD52 Trastuzumab increase the killing of B- cell lymphoma representative of Burkitt Lymphoma (see cell lines Raji and DAUDI).
FIGURE 7: Phosphoantigen activator Picostim plus anti-Her2Neu Herceptin increase the killing of Her2Neu carcinoma cells representative of Her2Neu breast cancer cells (see cell lines FKBR3).
FIGURE 8: Phosphoantigen activator Picostim plus anti-CD20 Rituximab activated the surface expression of lytic activity marker CD107a by γδ T cells in presence of B- cell lymphoma representative of Mantle Cell Lymphoma (see cell lines NCEB1).
FIGURE 9: Phosphoantigen activator Picostim plus anti-CD20 Rituximab activated the surface expression of lytic activity marker CD 107a by γδ T cells in presence of B- cell lymphoma representative of Follicular lymphoma (see cell lines RL - top panels - and KARPAS-422 - bottom histogram).
FIGURE 10: Phosphoantigen activator Picostim plus anti-CD20 Rituximab activated the surface expression of lytic activity marker CD107a by γδ T cells in presence of B-cell lymphoma representative of Burkitt Lymphoma (see cell lines Raji and DAUDI).
FIGURE 11: Phosphoantigen activator Picostim plus anti-CD52 Trastuzumab activated the surface expression of lytic activity marker CD107a by γδ T cells in presence of B-cell lymphoma representative of Mantle Cell Lymphoma (see cell lines ES-MOULT, GRANTA).
FIGURE 12: Phosphoantigen activator Picostim plus anti-CD52 Trastuzumab activated the surface expression of lytic activity marker CD107a by γδ T cells in presence of B-cell lymphoma representative of Follicular lymphoma (see cell lines RL).
FIGURE 13: Phosphoantigen activator Picostim plus anti-CD52 Trastuzumab increase the killing of B- cell lymphoma representative of Burkitt Lymphoma (see cell lines Raji and DAUDI).
FIGURE 14: Phosphoantigen activator Picostim plus anti-Her2Neu Herceptin increase the killing of Her2Neu carcinoma cells representative of Her2Neu breast cancer cells (see cell lines FKBR3).

### Detailed Description of the Invention

### COMBINATION OF THERAPEUTIC ANTIBODIES AND γδ T CELL ACTIVATOR COMPOUNDS

The present invention concerns a pharmaceutical composition comprising a γδ T cell activator compound and a therapeutic antibody. The pharmaceutical composition can further comprising a pharmaceutically carrier. It can also comprise other active agent. In particular, the composition can further comprise a cytokine, preferably an interleukin, more preferably IL-2. Indeed, IL-2 can provide improved in vivo expansion of γδ T cells. Furthermore, the compositions of this invention may further comprise or may be used in combination with other active agents or therapeutic programs such as chemotherapy or other immunotherapies, either simultaneously or sequentially. The present invention also concerns kits comprising a therapeutic antibody and a γδ T cell activator compound. In addition, the present invention concerns a product containing a γδ T cell activator compound and a therapeutic antibody as a combined preparation for simultaneous, separate or sequential use in the treatment of a disease. More particularly, the treatment of the disease requires the depletion of the targeted cells, preferably the diseased cells such as virally-infected cells, tumor cells, cells underlying an autoimmune disorder, or other pathogenic cells. Preferably, the disease is a cancer, infectious or immune disease. More preferably, the disease is selected from the group consisting of a cancer, an auto-immune disease, an inflammatory disease, and an infectious (e.g. bacterial or viral) disease. The disease also concerns a graft rejection, more particularly allograft rejection, and graft versus host disease (GVHD).

In addition, the present invention concerns a pharmaceutical composition comprising a therapeutic antibody and γδ T cells activated by a γδ T cell activator compound. γδ T cells activated by a γδ T cell activator compound can be prepared as described in the patent application US 2005/196385, the disclosure of which is incorporated herein by reference. The present invention also concerns kits comprising a therapeutic antibody and γδ T cells activated by a γδ T cell activator compound. The present invention concerns a product containing a therapeutic antibody and γδ T cells activated by a γδ T cell activator compound as a combined preparation for simultaneous, separate or sequential use in the treatment of a disease.

Compositions of this invention may comprise any pharmaceutically acceptable carrier or excipient, typically buffer, isotonic solutions, aqueous suspension, optionally supplemented with stabilizing agents, preservatives, etc. Typical formulations include a saline solution and, optionally, a protecting or stabilizing molecule, such as a high molecular weight protein (e.g., human serum albumin).

The invention also concerns the use of a γδ T cell activator compound and a therapeutic antibody for the preparation of a medicament for treating a disease. The present invention further concerns a method for treating a disease in a subject comprising administering a γδ T cell activator compound and a therapeutic antibody to the subject. The administration of the γδ T cell activator compound and the therapeutic antibody can be simultaneous, separate or sequential. The method can further comprise the administration of a cytokine, in particular of IL-2. More particularly, the treatment of the disease requires the depletion of the targeted cells, preferably the diseased cells such as virally-infected cells, tumor cells or other pathogenic cells.

The invention also concerns the use of a therapeutic antibody and γδ T cells activated by a γδ T cell activator compound for the preparation of a medicament for treating a disease. The present invention further concerns a method for treating a disease in a subject comprising administering a therapeutic antibody and γδ T cells activated by a γδ T cell activator compound to the subject. The administration of γδ T cells activated by a γδ T cell activator compound and the therapeutic antibody can be simultaneous, separate or sequential. The method can further comprise the administration of a cytokine, in particular of IL-2. More particularly, the treatment of the disease requires the depletion of the targeted cells, preferably the diseased cells such as virally-infected cells, tumor cells or other pathogenic cells. Preferably, the disease is a cancer, infectious or immune disease. More preferably, the disease is selected from the group consisting of a cancer, an auto-immune disease, an inflammatory disease, and an infectious (e.g. bacterial or viral) disease. The disease also concerns a graft rejection, more particularly allograft rejection, and graft versus host disease (GVHD).

The contemplated diseases include neoplastic proliferation of hematopoietic cells. Optionally, said diseases are selected from the group consisting of lymphoblastic leukemia, acute or chronic myelogenous leukemia, Hodgkin's lymphoma, Non-Hodgkin's lymphoma, myelodysplastic syndrome, multiple myeloma, and chronic lymphocytic leukemia. Said diseases also include ENT cancers, colorectal cancers, breast cancer, epithelial cancer. Said diseases include viral infection, such as CMV infection, and hepatitis B. Said diseases include inflammatory diseases, such as Crohn disease, rheumatoid arthritis, asthma, psoriasis, multiple sclerosis or diabetes. In particular, any disease listed in the table 1 can be treated.

According to the methods and compositions of the present invention, compounds, preferably γδ T cell activator compounds and therapeutic antibodies, are administered in an "efficient" or "therapeutically effective" amount.

In one embodiment, the method of treatment according to the present invention further comprises an additional step in which the activity or number of γδ T cells in the subject is assessed prior or subsequent to the administration of the γδ T cell activator compound. In another embodiment, the additional step involves i) obtaining γδ T cells from the subject prior to the administration; ii) incubating the γδ T cells in the presence of one or more target cells that are recognized by the therapeutic antibody, in the presence or absence of the γδ T cell activator compound; and iii) assessing the effect of the γδ T cell activator compound on the ability of the γδ T cells to deplete the target cells; wherein a detection that the γδ T cell activator compound enhances the ability of the γδ T cells to deplete the target cells indicates that the compound is suitable for use in the method, and that the method is suitable for use with the subject.

The present invention also concerns a method of killing target cells in a subject comprising administering a γδ T cell activator compound and a therapeutic antibody targeting the target cells to the subject. In particular, the target cells can be cancer cells, infected cells, antibody-coated cells. The present invention further concerns a method for increasing the killing of target cells in a subject comprising administering a γδ T cell activator compound to the subject, wherein a therapeutic antibody targeting the target cells is administered to the subject. The therapeutic antibody targeting the target cells can be administered before, simultaneously or after the γδ T cell activator compound. The present invention concerns in addition a method of killing target cells in a subject comprising administering a therapeutic antibody targeting the target cells and γδ T cells activated by a γδ T cell activator compound to the subject.

The invention also concerns the use of a γδ T cell activator compound (or activated γδ T cells) for increasing the efficiency of a treatment with a therapeutic antibody. In particular, the present invention concerns the use of a γδ T cell activator compound (or activated γδ T cells) for the preparation of a drug for increasing the efficiency of a treatment involving the administration of a therapeutic antibody in a subject, wherein the administration of said therapeutic antibody is administered to said subject prior to, simultaneously with, or following, a therapeutically-effective amount of a γδ T cell activator compound (or activated γδ T cells). The present invention also concerns a method for increasing in a subject the efficiency of a treatment with a therapeutic antibody, wherein a γδ T cell activator compound (or activated γδ T cells) is administered to the subject prior to, simultaneously with, or following the administration of a therapeutic antibody.

In one embodiment, the candidate compound enhances the ability of the therapeutic antibody to destroy the target cells by 50%, 60%, 70%, 80%, 90%, 100%, 200%, 300%, 400%, 500%, or more.

In one embodiment, the therapeutic antibody and the γδ T cell activator compound (or the activated γδ T cells) are administered into the subject simultaneously. In another embodiment, the γδ T cell activator compound (or the activated γδ T cells) is administered to the subject within several week (e.g. 2, 3, 4, 5, or 6 weeks), within one week, within 4 days, within 3 days or on the same day (e.g. within about 24 hours) of the administration of the therapeutic antibody. In a first embodiment, the γδ T cell activator compound (or the activated γδ T cells) is administered to the subject before the therapeutic antibody. In a second embodiment, the therapeutic antibody is administered to the subject before the γδ T cell activator compound (or the activated γδ T cells). The γδ T cell activator compound (or the activated γδ T cells) and the therapeutic antibody are administered so that the synergic effect can be obtained.

The present invention also comprises a method for reducing the dosage of a therapeutic antibody, e.g. an antibody that depletes a target cell, in particular by administering a γδ T cell activator compound (or activated γδ T cells). For example, co-administration of a therapeutic antibody and a γδ T cell activator compound (or activated γδ T cells) allows a lower dose of the therapeutic antibody to be used. Such antibodies can be used at a 20%, 30%, 40%, 50%, 60%, 70%, 80%, 90% lower dose than the recommended dose in the absence of the compound.

In an important embodiment of the invention, the use of the γδ T cell activator compound (or activated γδ T cells) can allow therapeutic efficacy to be achieved with reduced doses of therapeutic antibodies. The use (e.g., dosage, administration regimen) of therapeutic antibodies can be limited by side effects, e.g., in the case of rituximab, fever, headaches, wheezing, drop in blood pressure, and others. Accordingly, while in many patients a standard dose of the therapeutic antibodies will be administered in combination with γδ T cell activator compounds (or activated γδ T cells), thereby enhancing the efficacy of the standard dose in patients needing ever greater therapeutic efficacy, in other patients, e.g., those severely affected by side effects, the administration of the γδ T cell activator compounds (or activated γδ T cells) will allow therapeutic efficacy to be achieved at a reduced dose of therapeutic antibodies, thereby avoiding side effects. In practice, a skilled medical practitioner will be capable of determining the ideal dose and administrative regimen of the therapeutic antibody and the γδ T cell activator compound (or activated γδ T cells) for a given patient, e.g. the therapeutic strategy that will be most appropriate in view of the particular needs and overall condition of the patient. Numerous references are available to guide in the determination of proper dosages, for both the therapeutic antibodies and the γδ T cell activator compounds (or activated γδ T cells), e.g., Remington: The Science and Practice of Pharmacy, by Gennaro (2003), ISBN: 0781750253; Goodman and Gilmans The Pharmacological Basis of Therapeutics, by Hardman , Limbird & Gilman (2001), ISBN: 0071354697; Rawlins E. A., editor, "Bentley's Textbook of Pharmaceutics", London: Bailliere, Tindall and Cox, (1977); and others.

In one embodiment, a medical practitioner can gradually lower the amount of the therapeutic antibody given in conjunction with the administration of any of γδ T cell activator compound (or activated γδ T cells); either in terms of dosage or frequency of administration, and monitor the efficacy of the therapeutic antibody; e.g. monitor γδ T cell activity; monitor the presence of target cells in the patient, monitor various clinical indications, or by any other means, and, in view of the results of the monitoring, adjust the relative concentrations or modes of administration of the therapeutic antibodies and/or γδ T cell activator compound to optimize therapeutic efficacy and limitation of side effects.

Suitable doses of the γδ T cell activator compounds and/or therapeutic antibodies can also generally be determined in vitro or in animal models, e.g. in vitro by incubating various concentrations of a therapeutic antibody in the presence of target cells, γδ T cells, and varying concentrations of one or more γδ T cell activator compounds, and assessing the extent or rate of target cell depletion under the various conditions, using standard assays (e.g. as described in the examples section). Alternatively, varying dosages of the therapeutic antibodies can be given to animal models for diseases treatable with the antibodies (e.g. an animal model for NHL in the case of rituximab), along with varying dosages of the γδ T cell activator compounds, and the efficacy of the antibodies (e.g. as determined by any suitable clinical, cellular, or molecular assay or criterion) in treating the animals can be assessed.

The composition or product according to the present invention may be injected directly to a subject, typically by intra-venous, intra-peritoneal, intra-arterial, intra-muscular or transdermic route. Several monoclonal antibodies have been shown to be efficient in clinical situations, such as Rituxan (Rituximab) or Xolair (Omalizumab), and similar administration regimens (i.e., formulations and/or doses and/or administration protocols) may be used with the composition of this invention. The γδ T cell activator compounds (or activated γδ T cells) and therapeutic antibodies can be administered by the same route or by different routes.

Therefore, the invention provides a method for determining a therapeutically-effective, reduced dose of a therapeutic antibody, e.g., an antibody intended to cause the depletion of a target cell, the method comprising i) co-incubating a first concentration of the therapeutic antibody with target cells and γδ T cells in the absence of a γδ T cell activator compound; ii) co-incubating a second, lower concentration of the therapeutic antibody with target cells and with γδ T cells in the presence of a γδ T cell activator compound; iii) determining if the depletion of target cells observed in step ii) is as great as the depletion observed in step i). If it is observed that step ii) is as efficacious as step i), then the relative concentrations of the γδ T activator compound and the therapeutic antibody can be varied, and depletion observed, in order to identify different conditions that would be suitable for use in a given patient, e.g., maximizing target cell depletion, lowered dose of therapeutic antibody, or lowered dose of the compound, depending on the particular needs of the patient.

In another aspect, the present invention provides a method of selecting a γδ T cell activator compound for administration in combination with a therapeutic antibody, said method comprising: i) providing a candidate γδ T cell activator compound; ii) incubating the therapeutic antibody with target cells specifically recognized by the therapeutic antibody in the presence of γδ T cells and in the presence or absence of the candidate compound; and iii) assessing the effect of the candidate compound on the ability of the γδ T cells to deplete the target cells; wherein a detection that the candidate compound enhances the ability of the γδ T cells to deplete the target cells indicates that the candidate compound is suitable for use in the method.

Within the context of the present invention, a subject or patient includes any mammalian subject or patient, more preferably a human subject or patient.

### THERAPEUTIC ANTIBODIES

The present invention deals with the use of a γδ T cell activating compound in combination with therapeutic antibodies. Any of a large variety of therapeutic antibodies can be used in the present invention.

The term "antibody," as used herein, refers to polyclonal and monoclonal antibodies. Depending on the type of constant domain in the heavy chains, antibodies are assigned to one of five major classes: IgA, IgD, IgE, IgG, and IgM. Several of these are further divided into subclasses or isotypes, such as IgG1, IgG2, IgG3, IgG4, and the like. An exemplary immunoglobulin (antibody) structural unit comprises a tetramer. Each tetramer is composed of two identical pairs of polypeptide chains, each pair having one "light" (about 25 kDa) and one "heavy" chain (about 50-70 kDa). The N-terminus of each chain defines a variable region of about 100 to 110 or more amino acids that is primarily responsible for antigen recognition. The terms variable light chain (V_{L}) and variable heavy chain (V_{H}) refer to these light and heavy chains respectively. The heavy-chain constant domains that correspond to the different classes of immunoglobulins are termed "alpha," "delta," "epsilon," "gamma" and "mu," respectively. The subunit structures and three-dimensional configurations of different classes of immunoglobulins are well known. IgG and/or IgM are the preferred classes of antibodies employed in this invention, with IgG being particularly preferred, because they are the most common antibodies in the physiological situation, because they are most easily made in a laboratory setting, and because IgGs are specifically recognized by Fc gamma receptors. Preferably the antibody of this invention is a monoclonal antibody. Particularly preferred are humanized, chimeric, human, or otherwise-human-suitable antibodies.

Within the context of this invention, the term "therapeutic antibody or antibodies" designates more specifically any antibody that functions to target cells in a patient and optionally to deplete targeted cells in a patient. In particular, therapeutic antibodies specifically bind to antigens present on the surface of the target cells, e.g. tumor specific antigens present predominantly or exclusively on tumor cells. Preferably, therapeutic antibodies include human Fc portions, or are capable of interacting with human Fc receptors. Therapeutic antibodies can target cells by any means, e.g. ADCC or otherwise, and can be "naked," i.e. with no conjugated moieties, or they can be conjugated with compounds such as radioactive labels or toxins.

For the purposes of the present invention, a "humanized" antibody refers to an antibody in which the constant and variable framework region of one or more human immunoglobulins is fused with the binding region, e.g. the CDR, of an animal immunoglobulin. Such humanized antibodies are designed to maintain the binding specificity of the non-human antibody from which the binding regions are derived, but to avoid an immune reaction against the non-human antibody.

A "chimeric antibody" is an antibody molecule in which (a) the constant region, or a portion thereof, is altered, replaced or exchanged so that the antigen binding site (variable region) is linked to a constant region of a different or altered class, effector function and/or species, or an entirely different molecule which confers new properties to the chimeric antibody, e.g., an enzyme, toxin, hormone, growth factor, drug, etc.; or (b) the variable region, or a portion thereof, is altered, replaced or exchanged with a variable region having a different or altered antigen specificity. In preferred embodiments of the present invention, the chimeric antibody nevertheless maintains the Fc region of the immunoglobulin, preferably a human Fc region, thereby allowing interactions with human Fc receptors on the surface of target cells.

The present compounds can be used to enhance the ability of therapeutic antibodies to deplete target cells that express an antigen that is specifically recognized by the therapeutic antibodies. Accordingly, any disease or condition that is caused or exacerbated at least in part by cells that can be targeted by a therapeutic antibody can be treated using the herein-described methods. Specific examples of target cells include tumor cells, virus-infected cells, allogenic cells, pathological immunocompetent cells (e.g., B lymphocytes, T lymphocytes, antigen-presenting cells, etc.) involved in allergies, autoimmune diseases, allogenic reactions, etc., or even healthy cells (e.g., endothelial cells in an anti-angiogenic therapeutic strategy). Most preferred target cells within the context of this invention are tumor cells and virus-infected cells. The therapeutic antibodies may, for instance, mediate a cytotoxic effect or cell lysis, particularly by antibody-dependent cell-mediated cytotoxicity (ADCC).

Te therapeutic antibody has a human immunoglobulin constant domain (Fc) of the G1, G2, G3 or G4 subtype (e.g. an IgG1, IgG2, IgG3, IgG4), and/or bind to CD16. Each of these subtypes have been demonstrated in at least some case to be depleting. In preferred embodiments, the therapeutic antibody comprises a human Fc region of the G1 or G3 subtype, or a human Fc region of the G2 or G4 subtype in case where the Fc region or the antibody have been modified or so produced as to increase the ability of the therapeutic antibody to deplete a target cell. In another embodiment, the therapeutic antibody is an antibody or a fragment thereof. The therapeutic antibodies may be polyclonal or monoclonal. Preferably, the therapeutic antibody is a monoclonal antibody or fragment thereof. In one embodiment, the therapeutic antibodies can be antibody fragments or derivatives such as, inter alia, a Fab fragment, a Fab'2 fragment, a CDR and a ScFv. Preferably a fragment is an antigen-binding fragment. In one embodiment, the therapeutic antibody is a human, humanized or chimeric antibody or a fragment thereof. Essentially, any therapeutic antibody, whether "naked" or conjugated with a radiolabel, toxin, or other moiety, or whether full length or a fragment; or whether a true antibody or a modified derivative of an antibody, can be used. In another embodiment, the therapeutic antibody is not conjugated with a radioactive or toxic moiety.

The therapeutic antibodies may be produced by hybridomas or by recombinant cells engineered to express the desired variable and constant domains. The antibodies may be single chain antibodies or other antibody derivatives retaining the antigen specificity and the lower hinge region or a variant thereof. These may be polyfunctional antibodies, recombinant antibodies, humanized antibodies, fragments or variants thereof. Therapeutic antibodies which comprise an antibody fragment may also include but are not limited to bispecific antibodies; one example a suitable bispecific antibody comprises an antigen binding region specific for CD16 and an antigen binding region specific for a tumor antigen. Other antibody formats comprising fragments include recombinant bispecific antibody derivatives combining the binding regions of two different antibodies on a single polypeptide chain, also referred to as BiTE^{™} (Kufer P, et al TRENDS in Biotechnology 2004; 22 (5): 238-244; and Baeuerle et al, Current Opinion in Molecular Therapeutics 2003; 5(4): 413-419, the disclosures of which are incorporated herein by reference.

Therapeutic antibodies are generally specific for surface antigens, e.g., membrane antigens. Most preferred therapeutic antibodies are specific for tumor antigens (e.g., molecules specifically expressed by tumor cells), such as CD20, CD52, ErbB2 (or HER2/Neu), CD33, CD22, CD25, MUC-1, CEA, KDR, αVβ3, etc., particularly lymphoma antigens (e.g., CD20). The therapeutic antibodies have preferably human or non human primate IgG1 or IgG3 Fc portion, more preferably human IgG1.

Typical examples of therapeutic antibodies of this invention are rituximab, alemtuzumab and trastuzumab. Such antibodies may be used according to clinical protocols that have been authorized for use in human subjects. Additional specific examples of therapeutic antibodies include, for instance, epratuzumab, basiliximab, daclizumab, cetuximab, labetuzumab, sevirumab, tuvurimab, palivizumab, infliximab, omalizumab, efalizumab, natalizumab, clenoliximab, etc. Other examples of preferred therapeutic antibodies for use in accordance with the invention include anti-ferritin antibodies (US Patent Publication no. 2002/0106324), anti-p140 and anti-sc5 antibodies (WO 02/50122), the disclosures of each of the above reference being incorporated herein by reference. Other examples of therapeutic antibodies are listed in the following table, any of which (and others) can be used in the present methods. It will be appreciated that, regardless of whether or not they are listed in the following table or described elsewhere in the present specification, any antibody that can target cells, and optionally deplete the targeted cell, e.g. by ADCC, can benefit from the present methods, and that the following Table 1 is non exhaustive, neither with respect to the antibodies listed therein, nor with respect to the targets or indications of the antibodies that are listed.

**Table 1: Therapeutic antibodies**

| Ab specificity | DCI | Commercial name | Typical Indications |
|---|---|---|---|
| Anti-CD20 | rituximab | MabThera®, Rituxan® | NHL B |
| Anti-CD20 | | Zevalin | NHL |
| Anti-CD20 | | Bexocar | NHL |
| Anti-CD52 | alemtuzumab | CAMPATH-1H® | CLL, allograft |
| Anti-CD33 | | SMART-M195 | AML |
| Anti-CD33 | | Zamyl^{™} | Acute myeloid Leukemia |
| Anti-HLA-DR antigen | | SMART-ID10 | NHL |
| Anti-HLA-DR | | Remitogen^{™} | NHL B |
| Anti-CD22 | epratuzumab | LymphoCide^{™} | NHL B |
| Anti-HER2 | | MDX-210 | Prostate and other cancers |
| Anti-erbB2 (HER-2/neu) | trastuzumab | Herceptin®, | Metastatic breast cancer |
| Anti-CA125 | | OvaRex | Ovarian cancer |
| Anti-MUC1 | | TriAb | Metastatic breast cancer |
| Anti-MUC1 | | BravaRex | Metastatic cancers |
| Anti-PEM antigen | | Theragyn, Therex | Ovarian cancer, breast cancer |
| Anti-CD44 | bivatuzumab | | Head and neck cancer |
| Anti-gp72 | MAb, idiotypic 105AD7 | | colorectal cancer |
| Anti-EpCAM | Anti-EpCAM; MT201 | IS-IL2 | cancer |
| | | | |
| Anti-CD 18 | AMD Fab | | age-related macular degeneration |
| Anti-CD18 | Anti-CD18 | | Myocardial infarction |
| | | | |
| anti-nuC242 | nuC242-DMI | | Colorectal, gastric, and pancreatic cancer |
| Anti-EGFR | MAb425 | | cancer |
| Anti-EGFR | ABX-EGF, panitumumab | | Cancer |
| Anti-EGFR (HER-1, erbB1) | cetuximab | | ENT and colorectal Cancers |
| Anti-MUC-1 | | Therex® | Breast and epithelial cancers |
| Anti-CEA | | CEAVac | Colorectal cancer |
| Anti-CEA | labetuzumab | CEA-Cide^{™} | Solid tumors |
| Anti-αVβ3 | | Vitaxin | Leiomyosarcoma, colorectal and other cancers (anti-angiogenic) |
| | | | |
| anti-RSV protein | palivizumab | Synagis® | Viral diseases |
| Idem | | Numax^{™} | Idem |
| CMV | sevirumab | Protovir | CMV Infection |
| HBs | tuvirumab | Ostavir^{™} | Hepatitis B |
| Anti-CD25 | basiliximab | Simulect® | Prevention/treatment allograft rejection |
| Anti-CD25 | daclizumab | Zénapax® | Prevention/treatment allograft rejection |
| anti-TNF-α | infliximab | Remicade^{™} | Crohn disease, rheumatoid arthritis |
| anti-CD80 | IDEC-114 | | psoriasis |
| anti-IgE | | E-26 | Allergic asthma and rhinitis |
| anti-IgE | omalizumab | Xolair^{™} | Asthma |
| anti-IgE | Rhu-mAb E25 | | Allergy/asthma |
| anti-integrin αL (CD11a, LFA-1) | efalizumab | Xanelim^{™} | psoriasis |
| Anti-beta 2 integrin | LDP-01 | | Stroke, allograft rejection |
| anti-integrin αL (CD11a, LFA-1) | anti-CD 11a | | psoriasis |
| anti-CD4 | keliximab siplizumab MEDI-507 | | GVHD, psoriasis |
| Anti-CD4 | Zanolimimab/ HuMax-CD4 | | Cutaneous T cell lymphoma |
| Anti-anthrax protective antigen | MDX-1303 | Valortim® | Allograft rejection |
| Anti-C. difficile toxin B | MDX-1388 | | Allograft rejection |
| Anti-CD4 | OKT4A | | Allograft rejection |
| Anti-CD3 | OKT3 | | Allograft rejection |
| Anti-CD3 | SMART-aCD3 | | Autoimmune disease, allograft rejection, psoriasis |
| Anti-CD64 | | | anemia |
| anti-CD 147 | | | GvHD |
| anti-integrin α4 (α4β1-α4β7) | natalizumab | Antegren® | Multiple Sclerosis, Crohn |
| Anti-integrin β7 | | | Crohn, ulcerative colitis |
| Alpha 4 beta 7 | LDP-02 | | Ulcerative colitis |
| Anti-HLA-DR10 beta | | Oncolym | NHL |
| Anti-CD3 | | Nuvion | T cell malignancies |
| Anti-GD2 ganglioside | | Trigem | Metastatic melanoma and small cell lung cancer |
| Anti-SK-1 antigen | | | Colorectal and pancreatic carcinoma |
| anti-MICA or MICB | | | Cancers |
| Anti-RAET1/ULBP family, RAET1E, | | | Cancers |
| RAET1G | | | |
| anti-CD4* | clenoliximab | | |
| anti-IL-8 | ABX-IL8 | | psoriasis |
| Anti-VLA-4 | | Antegren | MS |
| Anti-CD40L | | Antova | SLE, allograft rejection |
| Anti-CD40L | IDEC-131 | | MS, SLE |
| Anti-E-selectin | CDP850 | | psoriasis |
| Anti-CD11/CD18 | Hu23F2G | | MS, stroke |
| Anti-ICAM-3 | ICM3 | | psoriasis |
| Anti-CBL | ABX-CBL | | GVHD |
| Anti-CD147 | | | |
| Anti-CD23 | IDEC-152 | | Asthma, allergies |
| Anti-CD25 | | Simulect | Allograft rejection |
| Anti-T1-ACY | ACY-110 | | Breast cancer |
| Anti-TTS | TTS-CD2 | | Pancreatic, renal cancer |
| Anti-TAG72 | AR54 | | Breast, ovarian, lung cancer |
| Anti-CA19.9 | GivaRex | | Colorectal, pancreatic, gastric |
| Anti-PSA | ProstaRex | | Prostate cancer |
| Anti-HMFG1 | R1550 | | Breast, gastric cancer |
| | pemtumomab | Theragyn | Gastric, ovarian cancer |
| Anti-hCG | CTP-16, CTP-21 | | Mutiple cancers |
| Anti collagen Types 1-V | HU177; HUIV26; XL313 | | Multiple cancers |
| Anti-CD46 | | Crucell/J&J | Mutiple cancers |
| Anti-17A-1 | Edrecolomab | Panorex | Colorectal cancer |
| Anti-HM1.24 | AHM | | Multiple myeloma |
| Anti-CD38 | Anti-CD38 | | Multiple myeloma |
| Anti-IL 15 Receptor | HuMax lymphoma | | Lymphoma |
| Anti-IL6 | B-E8 | | Lymphoma |
| Anti-TRAIL-R1 | TRM-1 | | Mutiple cancers |
| | | | |
| Anti-BlyS | Lymphostat | | Mutiple cancers |
| Anti-SCLC, CEA and DTPA | Pentacea | | Lung cancer |
| Anti-CD52 | CAMPATH | | Leukemia, Lymphoma |
| Anti-Lewis Y antigen | IGN311 | | Epithelial cancers |
| Anti-VE cadherin | E4G10 | | Mutiple cancers |
| Anti-CD56 | BB10901, huN901DC1 | | Colorectal, lung cancer |
| Anti-mertansine/mucine | Cantuzumab | | Colorectal, lung, pancreatic cancer |
| Anti-AFP | AFP-cide | | Liver cancer |
| Anti-CSAp | Mu-9 | | Colorectal cancer |
| Anti-CD30 | MDX-060 | | Melanoma, Hodgkins Disease |
| Anti-PSMA | MDX-070 | | Prostate cancer |
| Anti-CD15 | MDX-11 | | Leukemia |
| Anti-TAG72 | MDX-020 | | Colorectal cancer |
| Anti-CD19,CD3 bispecific | MT103 | | Lymphoma |
| Anti-mesothelin antigen | SS1-PE38 | | Brain and overian cancer, mesothelioma |
| Anti-DNA and histones | Cotara | | Colorectal, pancreatic, sarcoma, brain and other cancers |
| Anti-a5B1 integrin | Anti-a5 B1 | | Multiple cancers |
| Anti-p97 | SGN17/19 | | Melanoma |
| Anti-CD5 | Genimune | | Leukemia, lymphoma |

Therefore, the therapeutic antibody can be an antibody selected from the antibodies in Table 1 or an antibody binding the same antigen. The efficient amount of therapeutic antibodies administered to the subject can be between about 0.1 mg/kg and about 20 mg/kg. The efficient amount of antibody depends however of the form of the antibody (whole Ig, or fragments), affinity of the mAb and pharmacokinetics parameter that must be determined for each particular antibodies.

In a preferred embodiment, the antibody is the Rituximab. In a more particular embodiment, the antibody is rituximab, and said antibody is administered at a dosage of less than 375 mg/m² per week. In another embodiment, the antibody is Campath. In a more particular embodiment, the antibody is Campath, and the antibody is administered at a dosage of less than 90mg per week.

### γδ T LYMPHOCYTE ACTIVATORS

The term "γδT lymphocyte or cell activating compound" designates a molecule, preferably artificially produced, which can activate γδT lymphocytes (i.e., cause the activation and/or expansion thereof). Preferably, the γδ T cell activator compound is an non-peptide antigen, most preferably a compound comprising a pyrophosphate or biphosphonate moiety. In yet other embodiments, the compound is any other activator of γδ T cells, including compounds that act directly or indirectly (e.g. by activating directly an immune cell other than γδ T cells). More particularly, the γδT lymphocyte activating compound is a chemical compound capable of selectively activating Vγ9Vδ2 T lymphocytes. Selective activation of Vγ9Vδ2 T lymphocytes indicates that the compound has a selective action towards specific cell populations, and essentially does not activate other T cell sub-types, such as Vδ1 T cells. Such selectivity, as disclosed in the present application, suggests that preferred compounds can cause a selective or targeted activation of the proliferation or biological activity of Vγ9Vδ2 T lymphocytes.

Preferably a γδ T lymphocyte activator is a compound capable of regulating the activity of a γδ T cell in a population of γδ T cell clones in culture. The γδ T lymphocyte activator is capable of regulating the activity of a γδ T cell population of γδ T cell clones in a at millimolar concentration, preferably when the γδ T cell activator is present in culture at a concentration of less than 100, 10 or 1 mM. Regulating the activity of a γδ T cell can be assessed by any suitable means, preferably by assessing cytokine secretion, most preferably TNF-α secretion as described herein. Methods for obtaining a population of pure γδ T cell clones is described in Davodeau et al, (1993) and Moreau et al, (1986), the disclosures of which are incorporated herein by reference. Preferably the compound is capable of causing at least a 20%, 50% or greater increase in the number of γδ T cells in culture, or more preferably at least a 2-fold increase in the number of γδ T cells in culture.

The γδT lymphocyte or cell activating compound is preferably an non-peptide antigen. In yet other embodiments, the compound is any other activator of γδ T cells, including compounds that act directly or indirectly (e.g. by activating directly an immune cell other than γδ T cells).

γδ T lymphocyte activators useful in the present invention comprise the compounds of formula (I) : 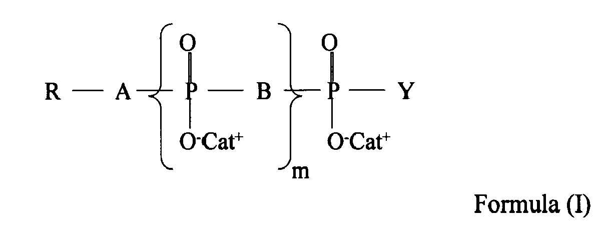
wherein Cat+ represents one (or several, identical or different) organic or mineral cation(s) (including proton);
m is an integer from 1 to 3;
B is O, NH, or any group capable to be hydrolyzed;
Y = O⁻Cat+, a C₁-C₃ alkyl group, a group -A-R, or a radical selected from the group consisting of a nucleoside, an oligonucleotide, a nucleic acid, an amino acid, a peptide, a protein, a monosaccharide, an oligosaccharide, a polysaccharide, a fatty acid, a simple lipid, a complex lipid, a folic acid, a tetrahydrofolic acid, a phosphoric acid, an inositol, a vitamin, a co-enzyme, a flavonoid, an aldehyde, an epoxyde and a halohydrin;
A is O, NH, CHF, CF₂ or CH₂; and,
R is a linear, branched, or cyclic, aromatic or not, saturated or unsaturated, C₁-C₅₀ hydrocarbon group, optionally interrupted by at least one heteroatom, wherein said hydrocarbon group comprises an alkyl, an alkylenyl, or an alkynyl, preferably an alkyl or an alkylene, which can be substituted by one or several substituents selected from the group consisting of : an alkyl, an alkylenyl, an alkynyl, an epoxyalkyl, an aryl, an heterocycle, an alkoxy, an acyl, an alcohol, a carboxylic group (-COOH), an ester, an amine, an amino group (-NH₂), an amide (-CONH₂), an imine, a nitrile, an hydroxyl (-OH), a aldehyde group (-CHO), an halogen, an halogenoalkyl, a thiol (-SH), a thioalkyl, a sulfone, a sulfoxide, and a combination thereof.

In a particular embodiment, the substituents as defined above are substituted by at least one of the substituents as specified above.

Preferably, the substituents are selected from the group consisting of : an (C₁-C₆)alkyl, an (C₂-C₆)alkylenyl, an (C₂-C₆)alkynyl, an (C₂-C₆)epoxyalkyl, an aryl, an heterocycle, an (C₁-C₆)alkoxy, an (C₂-C₆)acyl, an (C₁-C₆)alcohol, a carboxylic group (-COOH), an (C₂-C₆)ester, an (C₁-C₆)amine, an amino group (-NH₂), an amide (-CONH₂), an (C₁-C₆)imine, a nitrile, an hydroxyl (-OH), a aldehyde group (-CHO), an halogen, an (C₁-C₆)halogenoalkyl, a thiol (-SH), a (C₁-C₆)thioalkyl, a (C₁-C₆)sulfone, a (C₁-C₆)sulfoxide, and a combination thereof.

More preferably, the substituents are selected from the group consisting of : an (C₁-C₆)alkyl, an (C₂-C₆)epoxyalkyl, an (C₂-C₆)alkylenyl, an (C₁-C₆)alkoxy, an (C₂-C₆)acyl, an (C₁-C₆)alcohol, an (C₂-C₆)ester, an (C₁-C₆)amine, an (C₁-C₆)imine, an hydroxyl, a aldehyde group, an halogen, an (C₁-C₆)halogenoalkyl, and a combination thereof.

Still more preferably, the substituents are selected from the group consisting of : an (C₃-C₆)epoxyalkyl, an (C₁-C₃)alkoxy, an (C₂-C₃)acyl, an (C₁-C₃)alcohol, an (C₂-C₃)ester, an (C₁-C₃)amine, an (C₁-C₃)imine, an hydroxyl, an halogen, an (C₁-C₃)halogenoalkyl, and a combination thereof. and a combination thereof. Preferably, R is a (C₃-C₂₅)hydrocarbon group, more preferably a (C₅-C₁₀)hydrocarbon group.

In the context of the present invention, the term "alkyl" more specifically means a group such as methyl, ethyl, propyl, isopropyl, butyl, isobutyl, *tert*-butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, eicosyl, heneicosyl, docosyl and the other isomeric forms thereof. (C₁-C₆)alkyl more specifically means methyl, ethyl, propyl, isopropyl, butyl, isobutyl, tert-butyl, pentyl, hexyl and the other isomeric forms thereof. (C₁-C₃)alkyl more specifically means methyl, ethyl, propyl, or isopropyl.

The term "alkenyl" refers to an alkyl group defined hereinabove having at least one unsaturated ethylene bond and the term "alkynyl" refers to an alkyl group defined hereinabove having at least one unsaturated acetylene bond. (C₂-C₆)alkylene includes a ethenyl, a propenyl (1-propenyl or 2-propenyl), a 1- or 2- methylpropenyl, a butenyl (1-butenyl, 2-butenyl, or 3-butenyl), a methylbutenyl, a 2-ethylpropenyl, a pentenyl (1-pentenyl, 2-pentenyl, 3-pentenyl, 4-pentenyl), an hexenyl (1-hexenyl, 2-hexenyl, 3-hexenyl, 4-hexenyl, 5-hexenyl), and the other isomeric forms thereof. (C₂-C₆)alkynyl includes ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, or 5-hexynyl and the other isomeric forms thereof.

The term "epoxyalkyl" refers to an alkyl group defined hereinabove having an epoxide group. More particularly, (C₂-C₆)epoxyalkyl includes epoxyethyl, epoxypropyl, epoxybutyl, epoxypentyl, epoxyhexyl and the other isomeric forms thereof. (C₂-C₃)epoxyalkyl includes epoxyethyl and epoxypropyl.

The "aryl" groups are mono-, bi- or tri-cyclic aromatic hydrocarbons having from 6 to 18 carbon atoms. Examples include a phenyl, α-naphthyl, β-naphthyl or anthracenyl group, in particular.

"Heterocycle" groups are groups containing 5 to 18 rings comprising one or more heteroatoms, preferably 1 to 5 endocyclic heteroatoms. They may be mono-, bi- or tri-cyclic. They may be aromatic or not. Preferably, and more specifically for R₅, they are aromatic heterocycles. Examples of aromatic heterocycles include pyridine, pyridazine, pyrimidine, pyrazine, furan, thiophene, pyrrole, oxazole, thiazole, isothiazole, imidazole, pyrazole, oxadiazole, triazole, thiadiazole and triazine groups. Examples of bicycles include in particular quinoline, isoquinoline and quinazoline groups (for two 6-membered rings) and indole, benzimidazole, benzoxazole, benzothiazole and indazole (for a 6-membered ring and a 5-membered ring). Non aromatic heterocycles comprise in particular piperazine, piperidine, etc.

"Alkoxy" groups correspond to the alkyl groups defined hereinabove bonded to the molecule by an -O- (ether) bond. (C₁-C₆)alkoxy includes methoxy, ethoxy, propyloxy, butyloxy, pentyloxy, hexyloxy and the other isomeric forms thereof. (C₁-C₃)alkoxy includes methoxy, ethoxy, propyloxy, and isopropyloxy.

"Alcyl" groups correspond to the alkyl groups defined hereinabove bonded to the molecule by an -CO- (carbonyl) group. (C₂-C₆)acyl includes acetyl, propylacyl, butylacyl, pentylacyl, hexylacyl and the other isomeric forms thereof. (C₂-C₃)acyl includes acetyl, propylacyl and isopropylacyl.

"Alcohol" groups correspond to the alkyl groups defined hereinabove containing at least one hydroxyl group. Alcohol can be primary, secondary or tertiary. (C₁-C₆)alcohol includes methanol, ethanol, propanol, butanol, pentanol, hexanol and the other isomeric forms thereof. (C₁-C₃)alcohol includes methanol, ethanol, propanol and isopropanol.

"Ester" groups correspond to the alkyl groups defined hereinabove bonded to the molecule by an -COO- (ester) bond. (C₂-C₆)ester includes methylester, ethylester, propylester, butylester, pentylester and the other isomeric forms thereof. (C₂-C₃)ester includes methylester and ethylester.

"Amine" groups correspond to the alkyl groups defined hereinabove bonded to the molecule by an -N- (amine) bond. (C₁-C₆)amine includes methylamine, ethylamine, propylamine, butylamine, pentylamine, hexylamine and the other isomeric forms thereof. (C₁-C₃)amine includes methylamine, ethylamine, and propylamine.

"Imine" groups correspond to the alkyl groups defined hereinabove having a (-C=N-) bond. (C₁-C₆)imine includes methylimine, ethylimine, propylimine, butylimine, pentylimine, hexylimine and the other isomeric forms thereof. (C₁-C₃)imine includes methylimine, ethylimine, and propylimine.

The halogen can be Cl, Br, I, or F, more preferably Br or F.

"Halogenoalkyl" groups correspond to the alkyl groups defined hereinabove having at least one halogen. The groups can be monohalogenated or polyhalogenated containing the same or different halogen atoms. For example, the group can be an trifluoroalkyl (CF₃-R). (C₁-C₆)halogenoalkyl includes halogenomethyl, halogenoethyl, halogenopropyl, halogenobutyl, halogenopentyl, halogenohexyl and the other isomeric forms thereof. (C₁-C₃)halogenoalkyl includes halogenomethyl, halogenoethyl, and halogenopropyl.

"Thioalkyl" groups correspond to the alkyl groups defined hereinabove bonded to the molecule by an -S- (thioether) bond. (C₁-C₆)thioalkyl includes thiomethyl, thioethyl, thiopropyl, thiobutyl, thiopentyl, thiohexyl and the other isomeric forms thereof. (C₁-C₃)thioalkyl includes thiomethyl, thioethyl, and thiopropyl.

"Sulfone" groups correspond to the alkyl groups defined hereinabove bonded to the molecule by an -SOO- (sulfone) bond. (C₁-C₆)sulfone includes methylsulfone, ethylsulfone, propylsulfone, butylsulfone, pentylsulfone, hexylsulfone and the other isomeric forms thereof. (C₁-C₃)sulfone includes methylsulfone, ethylsulfone and propylsulfone.

"Sulfoxyde" groups correspond to the alkyl groups defined hereinabove bonded to the molecule by an -SO- (sulfoxide) group. (C₁-C₆)sulfoxide includes methylsulfoxide, ethylsulfoxide, propylsulfoxide, butylsulfoxide, pentylsulfoxide, hexylsulfoxide and the other isomeric forms thereof. (C₁-C₃)sulfoxide includes methylsulfoxide, ethylsulfoxide, propylsulfoxide and isopropylsulfoxide.

"Heteroatom" denotes N, S, or O.

"Nucleoside" includes adenosine, thymine, uridine, cytidine and guanosine.

In a particular embodiment, the hydrocarbon group is a cycloalkylenyl such as a cyclopentadiene or a phenyl, or an heterocycle such as a furan, a pyrrole, a thiophene, a thiazole, an imidazole, a triazole, a pyridine, a pyrimidine, a pyrane, or a pyrazine. Preferably, the cycloalkylenyl or the heterocycle is selected from the group consisting of a cyclopentadiene, a pyrrole or an imidazole. In a preferred embodiment, the cycloalkylenyl or the heterocycle is sustituted by an alcohol. Preferably, said alcohol is a (C₁-C₃)alcohol.

In an other embodiment, the hydrocarbon group is an alkylenyl with one or several double bonds. Preferably, the alkylenyl group has one double bond. Preferably, the alkylenyl group is a (C₃-C₁₀)alkylenyl group, more preferably a (C₄-C₇)alkylenyl group. Preferably, said alkylenyl group is substituted by at least one functional group. More preferably, the functional group is selected from the group consisting of an hydroxy, an (C₁-C₃)alkoxy, an aldehyde, an (C₂-C₃)acyl, or an (C₂-C₃)ester. In a more preferred embodiment, the hydrocarbon group is butenyl substituted by a group -CH₂OH. Optionally, said alkenyl group can be the isoform trans (E) or cis (Z), more preferably a trans isoform (E). In a most preferred embodiment, the alkylenyl group is the (E)-4-hydroxy-3-methyl-2-butenyl. In an other preferred embodiment, the alkylenyl group group is an isopentenyl, an dimethylallyl or an hydroxydimethylallyl.

In an additional embodiment, the hydrocarbon group is an alkyl group substituted by an acyl. More preferably, the hydrocarbon group is an (C₄-C₇)alkyl group substituted by an (C₁-C₃)acyl.

In a further preferred embodiment, R is selected from the group consisting of :
1) 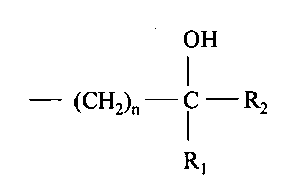 wherein n is an integer from 2 to 20, R₁ is a (C₁-C₃)alkyl group, and R₂ is an halogenated (C₁-C₃)alkyl, a (C₁-C₃)alkoxy-(C₁-C₃)alkyl, an halogenated (C₂-C₃)acyl or a (C₁-C₃)alkoxy-(C₂-C₃)acyl. Preferably, R₁ is a methyl or ethyl group, and R₂ is an halogenated methyl (-CH₂-X, X being an halogen), an halogenated (C₂-C₃)acetyl, or (C₁-C₃)alkoxy- acetyl. The halogenated methyl or acetyl can be mono-, di-, or tri-halogenated. Preferably, n is an integer from 2 to 10, or from 2 to 5. In a more preferred embodiment, n is 2. In a most preferred embodiment, n is 2, R₁ is a methyl and R₂ is an halogenated methyl, more preferably a monohalogenated methyl, still more preferably a bromide methyl. In a particularly preferred embodiment, n is 2, R₁ is a methyl, R2 is a methyl bromide. In a most preferred embodiment, R is 3-(bromomethyl)-3-butanol-1-yl.
2) 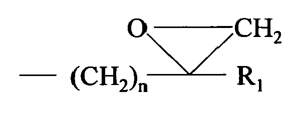 wherein n is an integer from 2 to 20, and R₁ is a methyl or ethyl group. Preferably, n is an integer from 2 to 10, or from 2 to 5. In a more preferred embodiment, n is 2 and R1 is a methyl.
3) 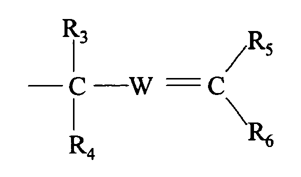 wherein R₃, R₄, and R₅ , identical or different, are a hydrogen or (C₁-C₃)alkyl group, W is -CH- or -N-, and R₆ is an (C₂-C₃)acyl, an aldehyde, an (C₁-C₃)alcohol, or an (C₂-C₃)ester. More preferably, R₃ and R₅ are a methyl and R₄ is a hydrogen. More preferably, R₆ is -CH₂-OH, -CHO, -CO-CH₃ or -CO-OCH₃. Optionally, the double-bond between W and C is in conformation trans (E) or cis (Z). More preferably, the double-bond between W and C is in conformation trans (E).

The group Y can allow to design a prodrug. Therefore, Y is enzymolabile group which can be cleaved in particular regions of the subject. The group Y can also be targeting group. In a preferred embodiment, Y is O⁻Cat+, a group -A-R, or a radical selected from the group consisting of a nucleoside, a monosaccharide, an epoxyde and a halohydrin. Preferably, Y is an enzymolabile group. Preferably, Y is O⁻Cat+, a group -A-R, or a nucleoside. In a first preferred embodiment, Y is O⁻Cat+. In a second preferred embodiment, Y is a nucleoside.

In a preferred embodiment, Cat⁺ is H⁺, Na⁺, NH₄⁺, K⁺, Li⁺, (CH₃CH₂)₃NH⁺.

In a preferred embodiment, A is O, CHF, CF₂ or CH₂. More preferably, A is O or CH₂.

In a preferred embodiment, B is O or NH. More preferably, B is O.

In a preferred embodiment, m is 1 or 2. More preferably, m is 1.

In one particular embodiment, synthetic γδT lymphocyte activators comprise the compounds of formula (II): 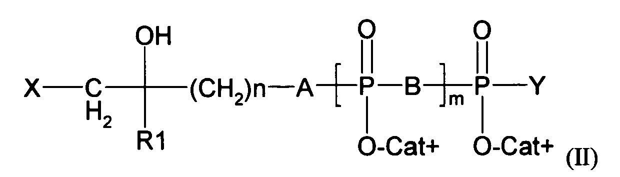 in which X is an halogen (preferably selected from I, Br and Cl), B is O or NH, m is an integer from 1 to 3, R1 is a methyl or ethyl group, Cat+ represents one (or several, identical or different) organic or mineral cation(s) (including the proton), and n is an integer from 2 to 20, A is O, NH, CHF, CF₂ or CH₂, and Y is O⁻Cat+, a nucleoside, or a radical -A-R, wherein R is selected from the group of 1), 2) or 3). Preferably, Y is O⁻ Cat+, or a nucleoside. More preferably, Y is O⁻Cat+. Preferably, R1 is a methyl. Preferably, A is O or CH₂. More preferably, A is O. Preferably, n is 2. Preferably, X is a bromide. Preferably, B is O. Preferably, m is 1 or 2. More preferably, m is 1.

For example, synthetic γδT lymphocyte activators comprise the compounds of formula (III) or (IV) : 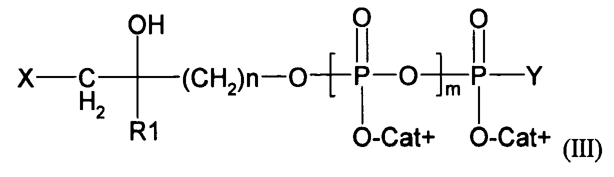 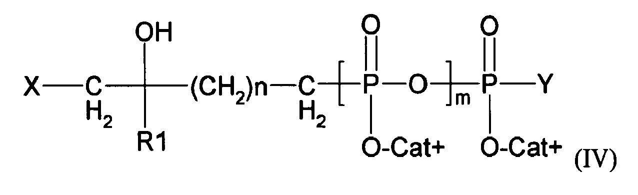 wherein X, R1, n, m and Y have the aforementioned meaning.

In one preferred embodiment, synthetic γδT lymphocyte activators comprise the compounds of formula (V): 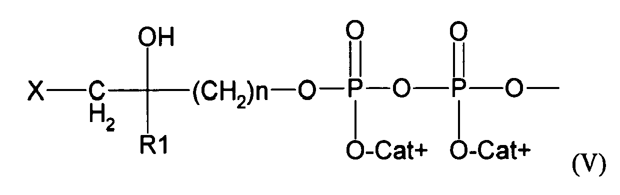 in which X is an halogen (preferably selected from I, Br and Cl), R1 is a methyl or ethyl group, Cat+ represents one (or several, identical or different) organic or mineral cation(s) (including the proton), and n is an integer from 2 to 20. Preferably, R1 is a methyl. Preferably, n is 2. Preferably, X is a bromide.

In a most preferred embodiment, synthetic γδT lymphocyte activators comprise the compound of formula (VI):

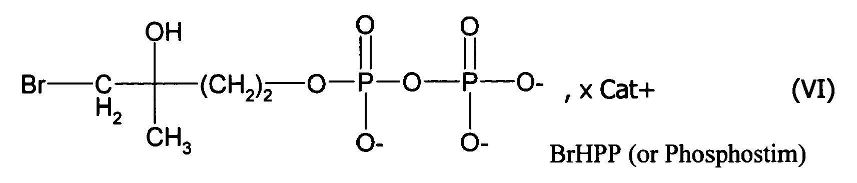

Preferably x Cat+ is 1 or 2 Na⁺.

In an other most preferred embodiment, synthetic γδT lymphocyte activators comprise the compound of formula (VII):

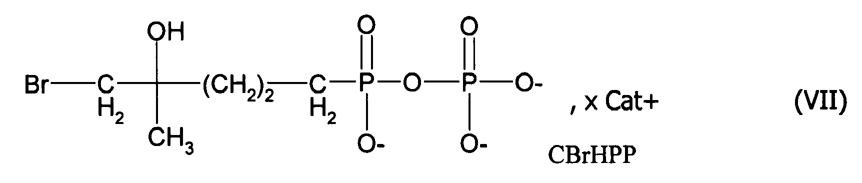

Preferably x Cat+ is 1 or 2 Na⁺.

In one particular embodiment, synthetic γδT lymphocyte activators comprise the compounds of formula (VIII): 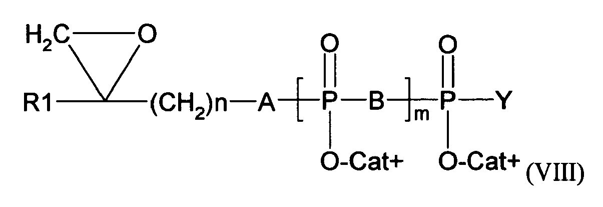 in which R1 is a methyl or ethyl group, Cat+ represents one (or several, identical or different) organic or mineral cation(s) (including the proton), B is O or NH, m is an integer from 1 to 3, and n is an integer from 2 to 20, A is O, NH, CHF, CF₂ or CH₂, and Y is O⁻Cat+, a nucleoside, or a radical -A-R, wherein R is selected from the group of 1), 2) or 3). Preferably, Y is O⁻Cat+, or a nucleoside. More preferably, Y is O⁻Cat+. Preferably, R1 is a methyl. Preferably, A is O or CH₂. More preferably, A is O. Preferably, n is 2. Preferably, B is O. Preferably, m is 1 or 2. More preferably, m is 1.

For example, synthetic γδT lymphocyte activators comprise the compounds of formula (IX) or (X) : 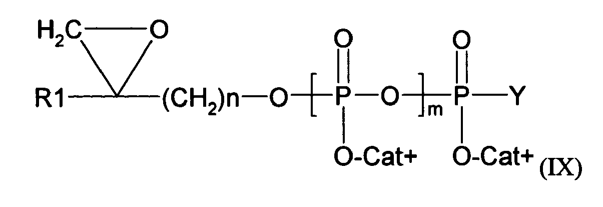 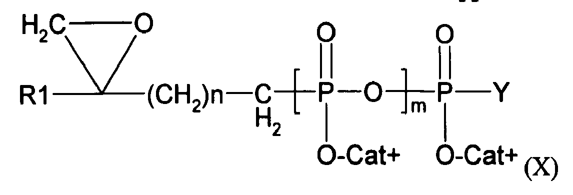 wherein R1, n, m and Y have the above mentioned meaning.

In one preferred embodiment, synthetic γδT lymphocyte activators comprise the compounds of formula (XI): 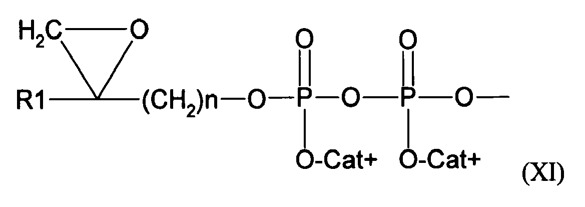 in which R1 is a methyl or ethyl group, Cat+ represents one (or several, identical or different) organic or mineral cation(s) (including the proton), and n is an integer from 2 to 20. Preferably, R1 is a methyl. Preferably, n is 2.

In a most preferred embodiment, synthetic γδT lymphocyte activators comprise the compound of formula (XI):

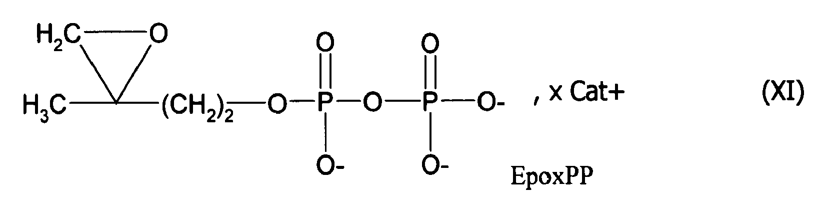

Preferably x Cat+ is 1 or 2 Na⁺.

In one particular embodiment, synthetic γδT lymphocyte activators comprise the compounds of formula (XII): 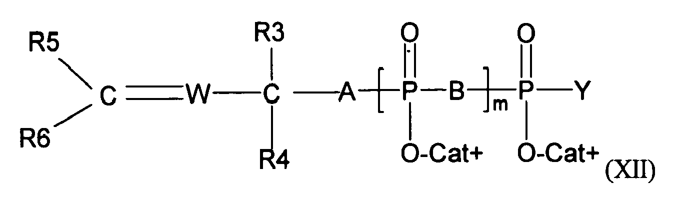 in which R₃, R₄, and R₅, identical or different, are a hydrogen or (C₁-C₃)alkyl group, W is -CH- or N-, R₆ is an (C₂-C₃)acyl, an aldehyde, an (C₁-C₃)alcohol, or an (C₂-C₃)ester, Cat+ represents one (or several, identical or different) organic or mineral cation(s) (including the proton), B is O or NH, m is an integer from 1 to 3, A is O, NH, CHF, CF₂ or CH₂, and Y is O⁻Cat+, a nucleoside, or a radical -A-R, wherein R is selected from the group of 1), 2) or 3). Preferably, Y is O⁻Cat+, or a nucleoside. More preferably, Y is O⁻ Cat+. Preferably, A is O or CH₂. More preferably, A is O. More preferably, R₃ and R₅ are a methyl and R₄ is a hydrogen. More preferably, R₆ is -CH₂-OH, -CHO, -CO-CH₃ or -CO-OCH₃. Preferably, B is O. Preferably, m is 1 or 2. More preferably, m is 1. Optionally, the double-bond between W and C is in conformation trans (E) or cis (Z). More preferably, the double-bond between W and C is in conformation trans (E).

For example, synthetic γδT lymphocyte activators comprise the compounds of formula (XIII) or (XIV) : 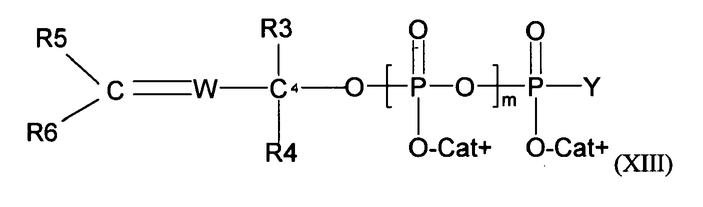 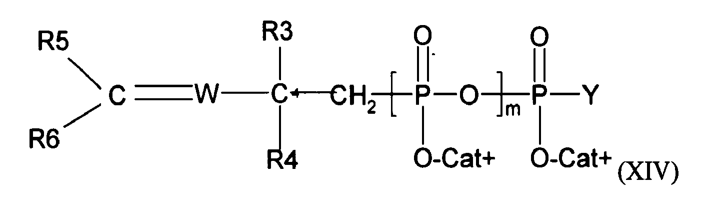 wherein R3, R4, R5, R6, W, m, and Y have the above mentionned meaning. Preferably, W is -CH-. Preferably, R3 and R4 are hydrogen. Preferably, R5 is a methyl. Preferably, R6 is -CH₂-OH.

In a most preferred embodiment, synthetic γδT lymphocyte activators comprise the compound of formula (XV):

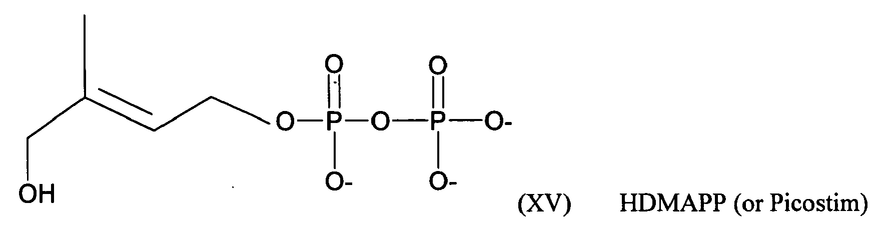

In an other most preferred embodiment, synthetic γδT lymphocyte activators comprise the compound of formula (XVI):

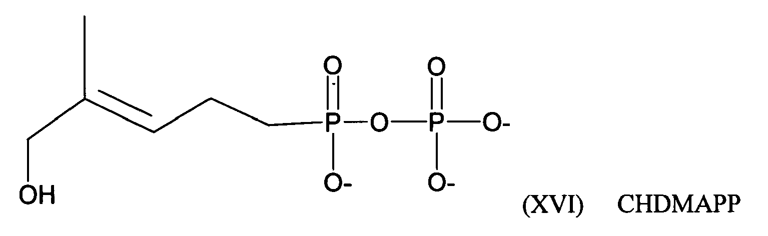

These compounds may be produced according to various techniques known per se in the art, some of which being disclosed in PCT Publications nos. WO 00/12516, WO 00/12519, WO 03/050128, and WO 03/009855, the disclosures of which are incorporated herein by reference.

In a most preferred embodiment, the synthetic γδT lymphocyte activating compound is selected from the group consisting of HDMAPP, CHDMAPP, Epox-PP, BrHPP and CBrHPP, more preferably HDMAPP, CHDMAPP, BrHPP and CBrHPP, still more preferably HDMAPP.

Alternatively, although potentially less efficient, other activators for use in the present invention are phosphoantigens disclosed in WO 95/20673, alkenyl pyrophosphates such as isopentenyl pyrophosphate (IPP) (US 5,639,653) and 3-methylbut-3-enyl pyrophosphonate (C-IPP). The disclosures of both references are incorporated herein by reference. Other compounds of interest include 2-methyl-3-butenyl-1-pyrophosphoric acid salts and other compounds in EP 1 153 928.

In particular, the γδ T cell activator can be HDMAPP, C-HDMAPP or N-HDMAPP.

The γδ T cell activator can also be an aminophosphonate, preferably an aminophosphonate of formula XVII : 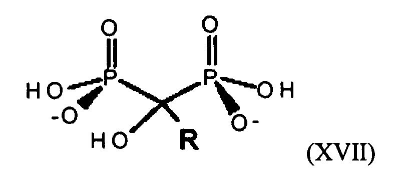 with R being a linear, branched, or cyclic, aromatic or not, saturated or unsaturated, C₁-C₅₀ hydrocarbon group, wherein said hydrocarbon group comprises an alkyl, an alkylenyl, or an alkynyl, preferably an alkyl or an alkylene, which is substituted by one or several substituents selected from the group consisting of : an amine, an amino group (-NH₂), an amide (-CONH₂), an imine, and a combination thereof.

In a preferred embodiment, R of formula XVII is a linear, branched, or cyclic, aromatic or not, saturated or unsaturated, C₁-C₁₀ hydrocarbon group, which is substituted by an amine, an amino group, a pyridine group, a pyrimidine group, a pyrrole group, an imidazole group, a pyrazole group, a triazole group.

In a still more preferred embodiment, R of formula XVII is selected from the group consisting of : 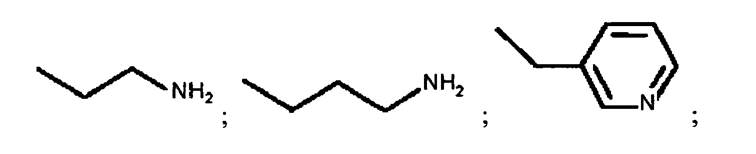 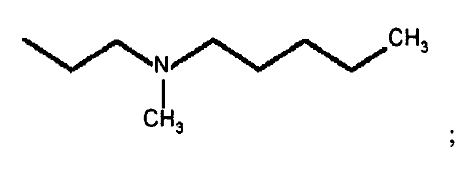 and

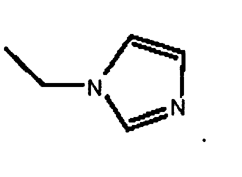

In particular, the γδ T cell activator can be selected from the group consisting of pamidronate, alendronate, ibandronate, risedronate and zoledronate.

Further aspects and advantages of this invention are disclosed in the following experimental section, which should be regarded as illustrative and not limiting the scope of this application.

### EXAMPLES

### Assay for cytolytic activity

A first experiment consists in the assessment of the tumoral cell death. Peripheral Vγ9δ2 T cells from healthy donors have been tested for lytic capacity towards several tumoral cell lines measured in standard cytotoxicity assay (4h ⁵¹Cr release). Tumoral cell lines were isotopically labelled with ⁵¹Cr. Release of ⁵¹Cr has been determined after 4 hours of co-culture. Specific lysis (expressed as percentage) is calculated using the standard formula [(experimental-spontaneous release / total-spontaneous release) x100].

Three experimental conditions have been used in order to compare tumoral cell death :
- tumoral cell lines + therapeutic antibody (Rituximab or Campath) with different concentration (100, 50 and 10 µg/ml);
- tumoral cell lines + activated γδ T cells by phosphantigen (BrHPP 100 nM, HDMAPP 20 nM or C-HDMAPP 20 nM) with different cell ratio (30:1, 10:1, 1:1);
- tumoral cell lines + activated γδ T cells by phosphantigen (BrHPP 100 nM, HDMAPP 20 nM or C-HDMAPP 20 nM) with different cell ratio (30:1, 10:1, 1: 1) + therapeutic antibody (Rituximab or Campath) at 10 µg/ml.

The experiments have been performed at least in triplicate. (*) and (**) mean highly significant, <1/100 and <1/1000, respectively.

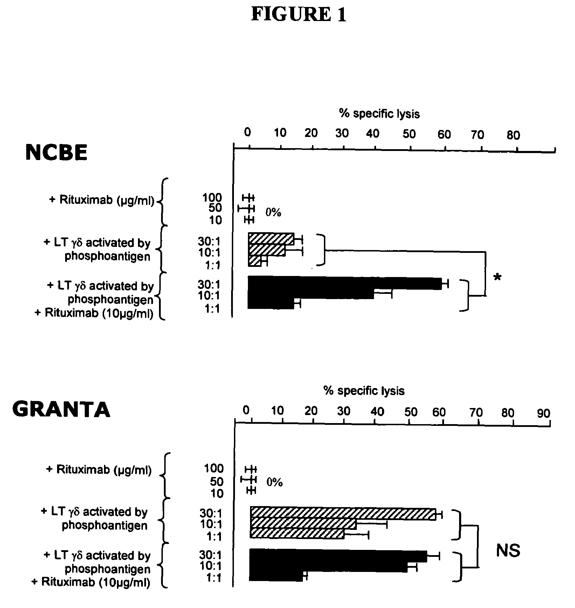

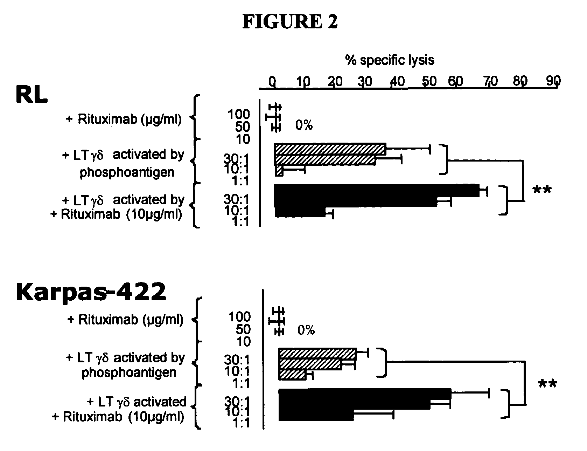

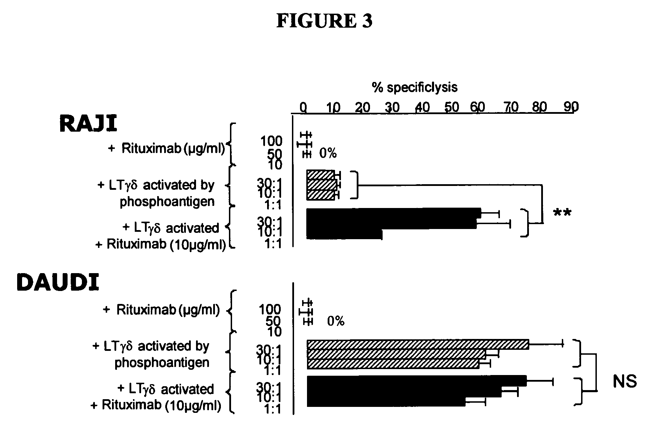

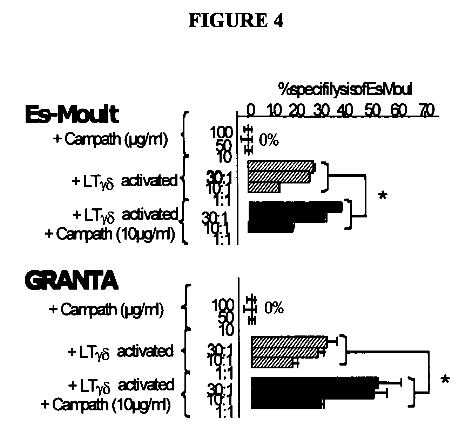

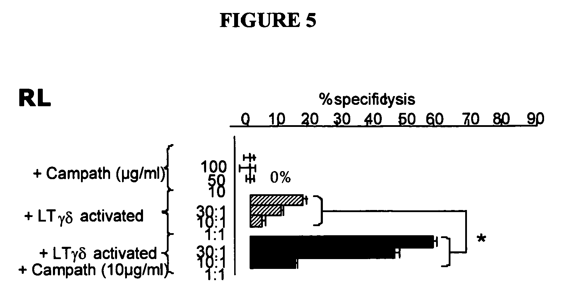

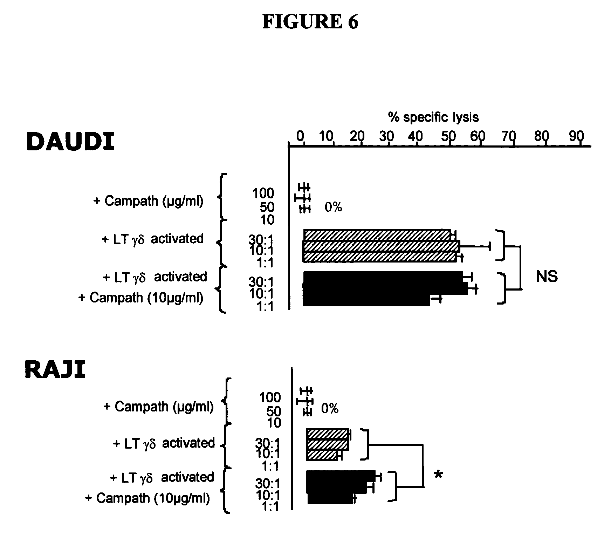

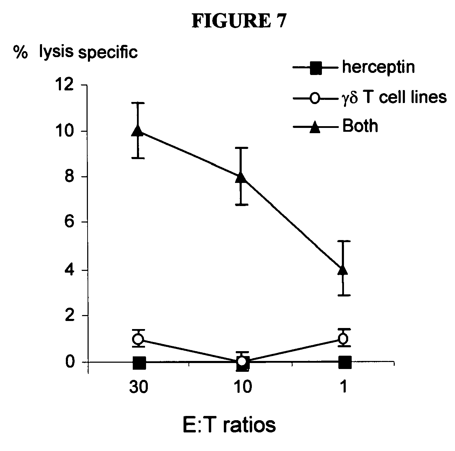

Tumoral cell lines tested are the following : NCBE, GRANTA, RL, Karpas-422, RAJI, DAUDI, and Es-Moult. The tested cell lines are indicated in the figures 1-7.

The results are shown in Figures 1-7 with C-HDMAPP. Same results have been observed with BrHPP and HDMAPP.

It has been observed for two different therapeutic antibodies that the tumoral cell death is higher by using a combination of a therapeutic antibody and a γδ T cell activator. Therefore, the therapeutic antibody and the γδ T cell activator have a synergic effect on the death of tumoral cells.

### Assay for cytolytic cells determination

A second experiment consists in the assessment of the cytotoxic capacity of Vγ9δ2 T cells. Tumoral cell lines were co-cultured as previously described with γδ T cells (therapeutic antibody alone 10µg/ml, activated Vγ982 T cells by phosphantigen alone, or both). The experiments determined the number of Vγ9δ2 T cells having cytotoxic activity. Vγ9δ2 T cellscells having cytotoxic capacity are known to express CD107a on their surface after coming into contact with a target cell susceptible to lysis. CD107a+ cells have been measured by flow cytometry.

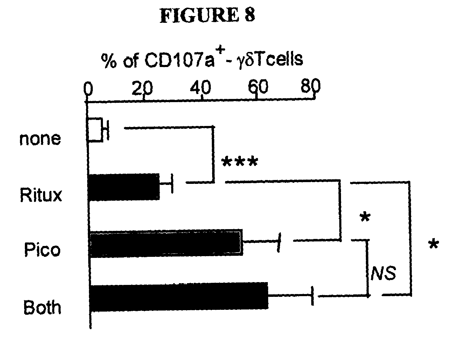

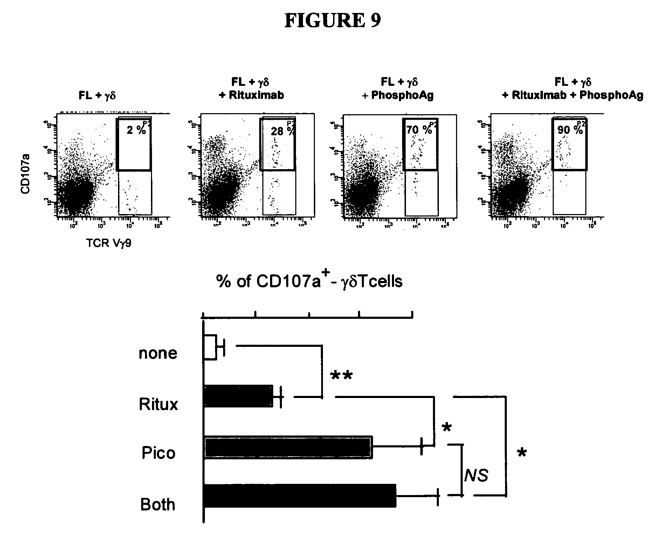

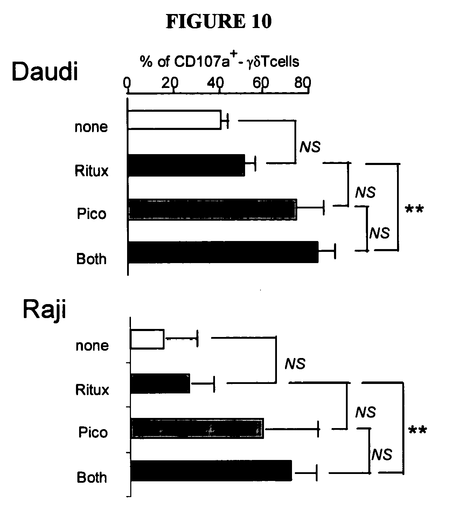

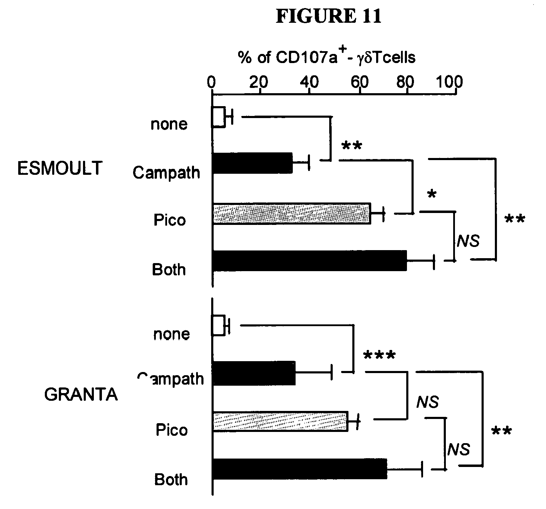

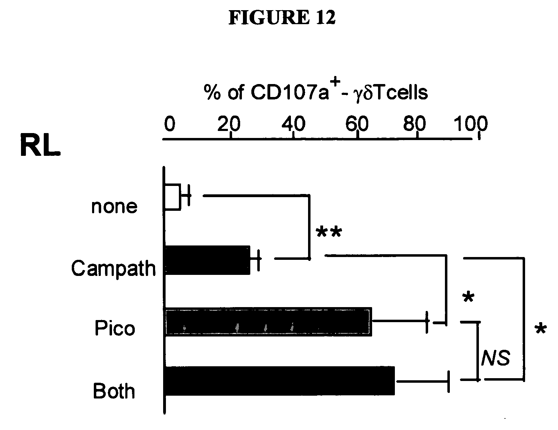

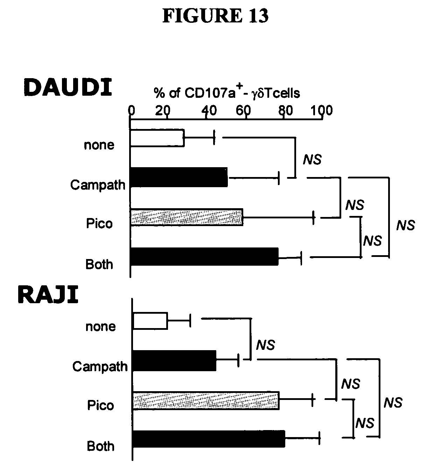

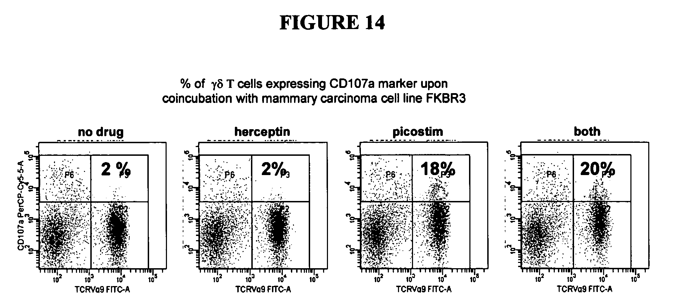

Tumoral cell lines tested are the following : NCBE, RL, Karpas-422, RAJI, DAUDI, GRANTA, FKBR3, and Es-Moult. The tested cell lines are indicated in Figures 8-14.

The results are shown in Figures 8-14 with C-HDMAPP. Same results have been observed with BrHPP and HDMAPP.

It has been observed for two different therapeutic antibodies that the number of cytotoxic Vγ9δ2 T cells is increased following a combination treatment with a therapeutic antibody and a γδ T cell activator.

## Claims

1. A pharmaceutical composition comprising a γδ T cell activator compound, a therapeutic antibody and a pharmaceutically acceptable carrier.

2. A product containing a γδ T cell activator compound and a therapeutic antibody as a combined preparation for simultaneous, separate or sequential use in the treatment of a disease.

3. Composition or product according to claim 1 or 2, wherein said therapeutic antibody targets diseased cells such as virally-infected cells, tumor cells, cells underlying an autoimmune disorder or other pathogenic cells.

4. Composition or product according to any one of claims 1-3, wherein the therapeutic antibody is a monoclonal, human, humanized or chimeric antibody or an antigen binding fragment thereof.

5. Composition or product according to any one of claims 1-4, wherein the therapeutic antibody is rituximab or campath.

6. Composition or product according to any one of claims 1-5, wherein the γδ T cell activator is a compound of formula (I) : 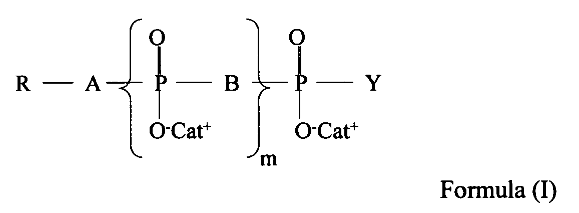
wherein Cat+ represents one (or several, identical or different) organic or mineral cation(s) (including proton);
m is an integer from 1 to 3;
B is O, NH, or any group capable to be hydrolyzed;
Y = O⁻Cat+, a C₁-C₃ alkyl group, a group -A-R, or a radical selected from the group consisting of a nucleoside, an oligonucleotide, a nucleic acid, an amino acid, a peptide, a protein, a monosaccharide, an oligosaccharide, a polysaccharide, a fatty acid, a simple lipid, a complex lipid, a folic acid, a tetrahydrofolic acid, a phosphoric acid, an inositol, a vitamin, a co-enzyme, a flavonoid, an aldehyde, an epoxyde and a halohydrin;
A is O, NH, CHF, CF₂ or CH₂; and,
R is a linear, branched, or cyclic, aromatic or not, saturated or unsaturated, C₁-C₅₀ hydrocarbon group, optionally interrupted by at least one heteroatom, wherein said hydrocarbon group comprises an alkyl, an alkylenyl, or an alkynyl, preferably an alkyl or an alkylene, which can be substituted by one or several substituents selected from the group consisting of : an alkyl, an alkylenyl, an alkynyl, an epoxyalkyl, an aryl, an heterocycle, an alkoxy, an acyl, an alcohol, a carboxylic group (-COOH), an ester, an amine, an amino group (-NH₂), an amide (-CONH₂), an imine, a nitrile, an hydroxyl (-OH), a aldehyde group (-CHO), an halogen, an halogenoalkyl, a thiol (-SH), a thioalkyl, a sulfone, a sulfoxide, and a combination thereof.

7. Composition or product according to claim 6, wherein the γδ T cell activator is a compound of formula (II): 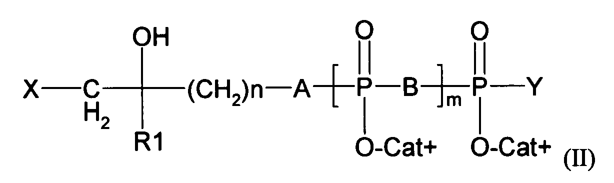 in which X is an halogen (preferably selected from I, Br and Cl), B is 0 or NH, m is an integer from 1 to 3, R1 is a methyl or ethyl group, Cat+ represents one (or several, identical or different) organic or mineral cation(s) (including the proton), and n is an integer from 2 to 20, A is O, NH, CHF, CF₂ or CH₂, and Y is O⁻Cat+.

8. Composition or product according to claim 7, wherein the γδ T cell activator is BrHPP, C-BrHPP or N-BrHPP.

9. Composition or product according to claim 6, wherein the γδ T cell activator is a compound of formula (XII): 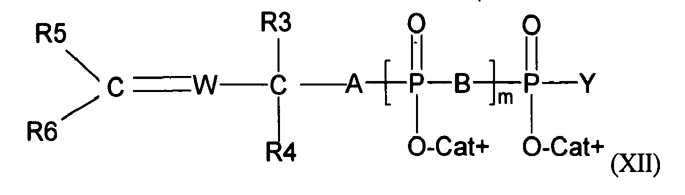 in which R₃, R₄, and R₅, identical or different, are a hydrogen or (C₁-C₃)alkyl group, W is -CH- or -N-, R₆ is an (C₂-C₃)acyl, an aldehyde, an (C₁-C₃)alcohol, or an (C₂-C₃)ester, Cat+ represents one (or several, identical or different) organic or mineral cation(s) (including the proton), B is O or NH, m is an integer from 1 to 3, A is O, NH, CHF, CF₂ or CH₂, and Y is O⁻Cat+.

10. Composition or product according to claim 9, wherein the γδ T cell activator is HDMAPP, C-HDMAPP or N-HDMAPP.

11. Composition or product according to any one of claims 1-5, wherein the γδ T cell activator is a compound of formula XVII : 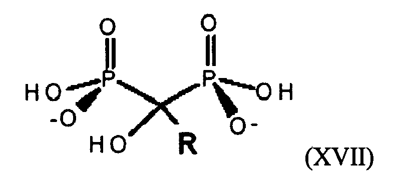 with R being a linear, branched, or cyclic, aromatic or not, saturated or unsaturated, C₁-C₅₀ hydrocarbon group, wherein said hydrocarbon group comprises an alkyl, an alkylenyl, or an alkynyl, preferably an alkyl or an alkylene, which is substituted by one or several substituents selected from the group consisting of : an amine, an amino group (-NH₂), an amide (-CONH₂), an imine, and a combination thereof.

12. Composition or product according to claim 11, wherein the γδ T cell activator can be selected from the group consisting of pamidronate, alendronate, ibandronate, risedronate and zoledronate.

13. Use of a γδ T cell activator compound and a therapeutic antibody for the preparation of a medicament for treating a disease.

14. Use of a γδ T cell activator compound for the preparation of a drug for increasing the efficiency of a treatment of a disease involving the administration of a therapeutic antibody in a subject, wherein the administration of said therapeutic antibody is administered to said subject prior to, simultaneously with, or following, a therapeutically-effective amount of a γδ T cell activator compound.

15. Use according to claim 13 or 14, wherein said therapeutic antibody targets the diseased cells such as virally-infected cells, tumor cells, cells underlying an autoimmune disorder or other pathogenic cells.

16. Use according to any one of claims 13-15, wherein the disease requires the depletion of the targeted cells, preferably the diseased cells such as virally-infected cells, tumor cells, cells underlying an autoimmune disorder or other pathogenic cells.

17. Use according to any one of claims 13-16, wherein the disease is selected from the group consisting of a cancer, an auto-immune disease, an inflammatory disease, and an infectious (e.g. bacterial or viral) disease.

18. Use according any one of claims 13-17, wherein the therapeutic antibody is a monoclonal, human, humanized or chimeric antibody or an antigen binding fragment thereof.

19. Use according any one of claims 13-18, wherein the therapeutic antibody is rituximab or campath.

20. Use according any one of claims 13-19, wherein the γδ T cell activator is a compound of formula (I) : 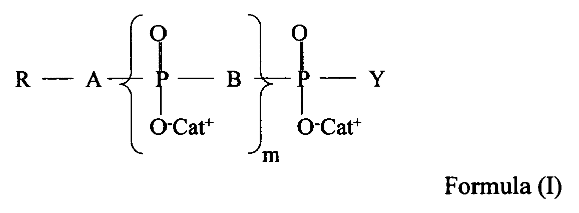
wherein Cat+ represents one (or several, identical or different) organic or mineral cation(s) (including proton);
m is an integer from 1 to 3;
B is O, NH, or any group capable to be hydrolyzed;
Y = O⁻Cat+, a C₁-C₃ alkyl group, a group -A-R, or a radical selected from the group consisting of a nucleoside, an oligonucleotide, a nucleic acid, an amino acid, a peptide, a protein, a monosaccharide, an oligosaccharide, a polysaccharide, a fatty acid, a simple lipid, a complex lipid, a folic acid, a tetrahydrofolic acid, a phosphoric acid, an inositol, a vitamin, a co-enzyme, a flavonoid, an aldehyde, an epoxyde and a halohydrin;
A is O, NH, CHF, CF₂ or CH₂; and,
R is a linear, branched, or cyclic, aromatic or not, saturated or unsaturated, C₁-C₅₀ hydrocarbon group, optionally interrupted by at least one heteroatom, wherein said hydrocarbon group comprises an alkyl, an alkylenyl, or an alkynyl, preferably an alkyl or an alkylene, which can be substituted by one or several substituents selected from the group consisting of : an alkyl, an alkylenyl, an alkynyl, an epoxyalkyl, an aryl, an heterocycle, an alkoxy, an acyl, an alcohol, a carboxylic group (-COOH), an ester, an amine, an amino group (-NH₂), an amide (-CONH₂), an imine, a nitrile, an hydroxyl (-OH), a aldehyde group (-CHO), an halogen, an halogenoalkyl, a thiol (-SH), a thioalkyl, a sulfone, a sulfoxide, and a combination thereof.

21. Use according to claim 20, wherein the γδ T cell activator is a compound of formula (II): 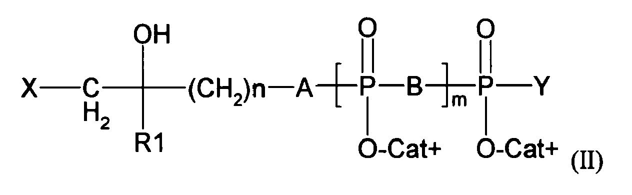 in which X is an halogen (preferably selected from I, Br and Cl), B is O or NH, m is an integer from 1 to 3, R1 is a methyl or ethyl group, Cat+ represents one (or several, identical or different) organic or mineral cation(s) (including the proton), and n is an integer from 2 to 20, A is O, NH, CHF, CF₂ or CH₂, and Y is O⁻Cat+.

22. Use according to claim 21, wherein the γδ T cell activator is BrHPP, C-BrHPP or N-BrHPP.

23. Use according to claim 20, wherein the γδ T cell activator is a composition comprising a compound of formula (XII): 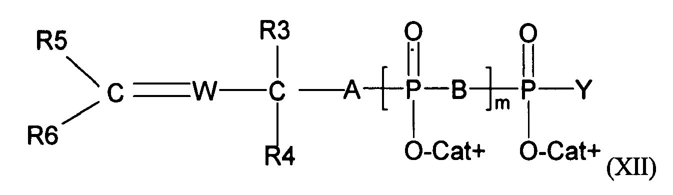 in which R₃, R₄, and R₅, identical or different, are a hydrogen or (C₁-C₃)alkyl group, W is -CH- or -N-, R₆ is an (C₂-C₃)acyl, an aldehyde, an (C₁-C₃)alcohol, or an (C₂-C₃)ester, Cat+ represents one (or several, identical or different) organic or mineral cation(s) (including the proton), B is O or NH, m is an integer from 1 to 3, A is O, NH, CHF, CF₂ or CH₂, and Y is O⁻Cat+.

24. Use according to claim 23, wherein the γδ T cell activator is HDMAPP, C-HDMAPP or N-HDMAPP.

25. Use according any one of claims 13-19, wherein the γδ T cell activator is a compound of of formula XVII : 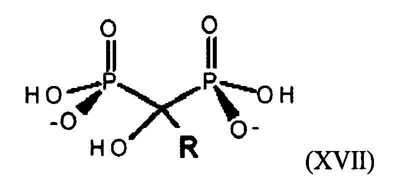 with R being a linear, branched, or cyclic, aromatic or not, saturated or unsaturated, C₁-C₅₀ hydrocarbon group, wherein said hydrocarbon group comprises an alkyl, an alkylenyl, or an alkynyl, preferably an alkyl or an alkylene, which is substituted by one or several substituents selected from the group consisting of : an amine, an amino group (-NH₂), an amide (-CONH₂), an imine, and a combination thereof.

26. Use according to claim 25, wherein the γδ T cell activator can be selected from the group consisting of pamidronate, alendronate, ibandronate, risedronate and zoledronate.
